(19) Europäisches Patentamt
European Patent Office
Office européen des brevets

(11) Publication number: **0 634 404 A1**

# (12) EUROPEAN PATENT APPLICATION

(21) Application number: **94110570.2**

(22) Date of filing: **07.07.94**

(51) Int. Cl.[6]: **C07D 237/32**, C07D 237/30, C07D 405/04, A01N 43/58, C07C 251/86

(30) Priority: **13.07.93 US 90726**
**13.07.93 US 90718**

(43) Date of publication of application:
**18.01.95 Bulletin 95/03**

(84) Designated Contracting States:
**AT BE CH DE DK ES FR GB GR IE IT LI LU NL PT SE**

(71) Applicant: **RHONE POULENC AGRICULTURE LTD.**
**Fyfield Road**
**Ongar,**
**Essex CM5 OHW (GB)**

(72) Inventor: **Bowden, Keith**
**RHONE POULENC AGRICULTURE LTD.**
**Fyfield Road**
**Ongar, Essex (GB)**
Inventor: **Bushey, Dean F**
**5336 Cherrycrest Ct.**
**Raleigh, NC 27609 (US)**
Inventor: **D'Silva, Themistocles**
**635 Kensington Drive**
**Chapel Hill**
**North Carolina27514 (US)**
Inventor: **Gant, Daniel B.**
**9 Greystone Ct.**
**Durham, NC 27713 (US)**
Inventor: **Herman, Nancy D.**
**106 Kirkcaldy Road**
**Cary, NC 27511 (US)**
Inventor: **Pettit, Simon Neil**
**Fyfield Road**
**Ongar, Essex (GB)**
Inventor: **Ray, Nicholas Ch.**
**RHONE POULENC AGRICULTURE LTD.**
**Fyfield Road**
**Ongar, Essex (GB)**
Inventor: **Smith, Philip Henry G.**
**RHONE POULENC AGRICULTURE LTD.**
**Fyfield Road**
**Ongar, Essex (GB)**

(74) Representative: **Brachotte, Charles et al**
**RHONE-POULENC AGROCHIMIE**
**Département Propriété Industrielle**
**B.P. 9163**
**F-69263 Lyon Cedex 09 (FR)**

## (54) Phtalazin derivatives and their use as pesticides.

(57) The invention provides compounds of formula:

EP 0 634 404 A1

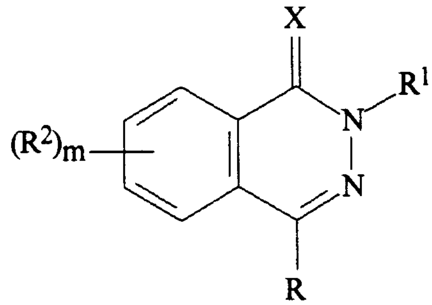

(I)

wherein the symbols are as defined in the description, which possess utility as pesticides (e.g. herbicides and insecticides).

## Background of the invention.

This invention relates to novel phthalazin-1-ones and phthalazin-1-thiones, compositions containing them, processes for their preparation; their use as pesticides, in particular as herbicides and/or insecticides; and intermediates used in their synthesis.

## Discussion of Prior art

A number of phthalazinone derivatives are described in the literature. For example, DE-A-2632656 discloses processes for preparing certain 4-unsubstituted phthalazin-1-ones. GB-A-2030136 describes 6,8-dimethyl-7-alkoxycarbonyl-1-phthalazinones having pharmaceutical properties. DE-A-2531776 relates to the use of 1-phthalazinones as azo-dyes, as does US Patent No. 3,880,881. WO-A-93/07146 discloses benzo and pyridopyridazinones having pharmaceutical activity as phosphodiesterase (PDE) IV enzyme inhibitors, WO-A-91/12251 relates to phthalazinones for use as antiasthmatic agents, and US Patent No. 3,694,442 discloses 2-(3-chloropropyl)-phthalazinone derivatives useful as intermediates in the synthesis of pharmaceutically active compounds.

In addition, a number of Chemical Abstract references [CA114 (1), 6413f, 1991; CA111(1), 7325d; CA111(28), 39285g, 1989; CA105(5), 4273n, 1986; CA83(28), 178862j, 1975; CA106(28), 156383v, 1987; CA97(13), 109949v, 1982; and CA116(28), 235555n, 1992] disclose the synthesis of certain 1-phthalazinones. None of these references disclose or suggest any use of 1-phthalazinones or thiones as pesticides. EP-A-0478195 discloses certain pyridazinones useful as fungicides.

## Description of the invention.

The present invention provides a method of controlling the growth of pests at a locus which comprises applying to the locus a pesticidally effective amount of a phthalazin-1-one or phthalazin-1-thione derivative of formula I:

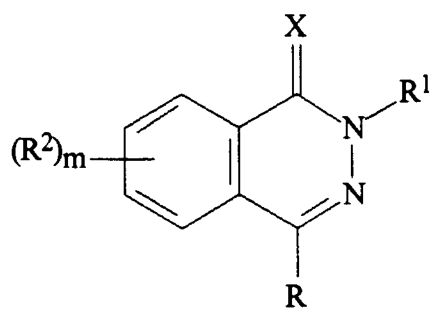

(I)

wherein:

R represents:-

the hydrogen atom;

a straight- or branched chain alkyl, alkenyl or alkynyl group containing up to six carbon atoms optionally substituted by one or more halogen atoms;

a group $-(-CR^3R^4)_n-(phenyl)-(R^{5a})_q$;

a group $-(-CR^3R^4)_nHet$;

a group $-(-CR^3R^4)_n-Ar$, wherein Ar represents phenyl or pyridyl optionally substituted by one or more groups $R^{5a}$ and wherein two substituents on adjacent positions of the ring, together with the two atoms to which they are attached, form a 5- to 7-membered alicyclic ring (which is optionally unsaturated) or an aromatic ring, optionally containing one or more heteroatoms (preferably selected from oxygen, sulphur and nitrogen, it being understood that a sulphur atom, where present, may be in the form of a group -SO- or $-SO_2-$), wherein the alicyclic or aromatic ring is optionally substituted by one or more groups $R^{51}$ which may be the same or different;

$R^1$ represents:-

a straight- or branched chain alkyl, alkenyl or alkynyl group containing up to six carbon atoms optionally

substituted by one or more halogen atoms;

a group $-(-CR^3R^4)_n$-(phenyl)-$(R^{5b})_q$;

a group $-(-CR^3R^4)_n$Het;

a group $-(-CR^3R^4)_n$-Ar, wherein Ar represents phenyl or pyridyl optionally substituted by one or more groups $R^{5b}$ and wherein two substituents on adjacent positions of the ring, together with the two atoms to which they are attached, form a 5- to 7-membered alicyclic ring (which is optionally unsaturated) or an aromatic ring, optionally containing one or more heteroatoms (preferably selected from oxygen, sulphur and nitrogen, it being understood that a sulphur atom, where present, may be in the form of a group -SO- or $-SO_2-$), wherein the alicyclic or aromatic ring is optionally substituted by one or more groups $R^{51}$ which may be the same or different;

provided that R represents a group $-(-CR^3R^4)_n$-(phenyl)-$(R^{5a})$q and/or $R^1$ represents a group $-(-CR^3R^4)_n$-(phenyl)-$(R^{5b})_q$ wherein n is zero;

$R^2$ represents:-

a group $R^5$;

or phenyl optionally substituted by from one to five groups $R^5$ which may be the same or different;

X represents oxygen or sulphur;

m represents zero or an integer from one to four;

where m is greater than one the groups $R^2$ may be the same or different;

$R^3$ and $R^4$, which may be the same or different, each represents the hydrogen atom or a straight- or branched- chain alkyl group containing up to four carbon atoms optionally substituted by one or more halogen atoms;

$R^5$, $R^{5a}$ and $R^{5b}$, which may be the same or different, each represents:-

a halogen atom;

a straight- or branched- chain alkyl, alkenyl or alkynyl group containing up to six carbon atoms optionally substituted by one or more halogen atoms; or

a group selected from cyano, nitro, $-CO_2R^6$, $-S(O)_pR^6$, $-NR^3R^4$, $-COR^6$, $-S(O)_pR^7$, $-CO_2R^7$, $-OR^7$, $-CONR^3R^4$, $-OSO_2R^7$, $-OSO_2R^8$, $-OCH_2R^7$, $-N(R^3)COR^8$, $-N(R^3)SO_2R^8$, $-N(R^3)SO_2R^7$, $-SO_2NR^3R^4$, $-Si(R^8)_3$ and $-OR^6$;

n represents zero one or two; where n is two the groups $-(-CR^3R^4)-$ may be the same or different;

q represents zero or an integer from one to five; where q is greater than one the groups $R^{5a}$ and/or $R^{5b}$ may be the same or different;

$R^{51}$ is as hereinbefore defined for $R^5$ or represents =O or =S;

Het represents a 5- or 6- membered heterocycle containing from 3 to 5 carbon atoms in the ring and from 1 to 3 heteroatoms in the ring selected from nitrogen, sulphur and oxygen (e.g. pyridyl, pyrimidyl, thienyl or pyrazolyl) optionally substituted by one or more groups $R^5$ which may be the same or different;

$R^6$ represents the hydrogen atom or a straight- or branched-chain alkyl group containing up to six carbon atoms optionally substituted by one or more halogen atoms;

p represents zero, one or two;

$R^7$ represents phenyl optionally substituted by from one to five groups which may be the same or different selected from halogen, nitro, cyano, $R^6$ and $-OR^6$;

$R^8$ represents a straight- or branched- chain alkyl group containing up to six carbon atoms optionally substituted by one or more halogen atoms;

or an agriculturally acceptable salt thereof.

In certain cases the substituents R, $R^1$, $R^2$, $R^3$, $R^4$, $R^5$, $R^{5a}$, $R^{5b}$, $R^6$ and $R^8$ contribute to optical and/or stereoisomerism. All such forms are embraced by the present invention.

By the term "agriculturally acceptable salts" is meant salts the cations or anions of which are known and accepted in the art for the formation of salts for agricultural or horticultural use. Preferably the salts are water soluble. Suitable salts formed by compounds of formula I which are acidic, e.g. compounds containing a carboxy group, with bases include alkali metal (e.g. sodium and potassium) salts, alkaline earth metal (e.g. calcium and magnesium) salts and ammonium (e.g. diethanolamine, triethanolamine, octylamine, dioctylamine and morpholine) salts.

Suitable acid addition salts, formed by compounds of formula I containing an amino group, include salts with inorganic acids, for example hydrochlorides, sulphates, phosphates and nitrates and salts with organic acids, for example acetic acid.

It is to be understood that where reference is made in the specification to the compounds of formula I, such reference is intended to include salts where the context so permits.

Where the group Ar represents optionally substituted phenyl or pyridyl with two substituents on adjacent positions of the ring forming a 5- to 7- membered alicyclic ring (which is optionally unsaturated) or

an aromatic ring, which contains one or more heteroatoms in the ring, generally there will be from one to three heteroatoms. Examples of the group Ar include optionally substituted methylenedioxybenzene, 2-mercaptobenzimidazole, 2-hydroxybenzimidazole, 2,3-dihydrobenzofuran, 1,3-benzoxathiazole, 1,2-benzoxathiazole, (3H)-1,2-benzisothiazole-1,1-dioxide, 1,2,5-benzothiadiazole-1,1-dioxide, indoline, benzofuroxan and 2,3-dihydrobenzo[b]thiophene.

Furthermore, where Ar represents pyridyl optionally substituted by one or two groups $R^5$, the two substituents on adjacent atoms of the pyridyl ring which form a 5- to 7- membered alicyclic or aromatic ring may be attached to two carbon atoms or to a carbon and nitrogen atom of the pyridyl ring.

A number of the compounds of formula I are novel and according to a feature of the invention there are provided phthalazin-1-one and phthalazin-1-thione derivatives of formula I as hereinbefore defined with the exclusion of the following compounds:

(a) X is oxygen, m is zero, $R^1$ is phenyl and R is 4-$N(CH_3)_2$-phenyl;

(b) X is oxygen, R is alkyl or hydrogen and $(R^2)_m$ is 6,8-dimethyl-7-alkylcarboxy, 5-nitro-6-methyl-7-alkylcarboxy or 5-nitro-6,8-dimethyl-7-alkylcarboxy;

(c) X is oxygen, m is zero, $R^1$ is selected from the group consisting of methyl, phenyl, 4-chlorophenyl and 3-chlorophenyl and R is phenyl substituted in the 3,4 or 5- position by $-NH_2$ and optionally bearing one or two additional substituents selected from alkyl, halogen, alkoxy, hydroxy, phenoxy phenylsulphonamide and nitro;

(d) X is oxygen and $R^1$ is $-(CH_2)_n$Het wherein n is 2;

(e) $R^1$ is phenyl and R is $-(CR^3R^4)_n$Het;

(f) X is oxygen, m is zero, $R^1$ is 4-pyridylmethyl, benzyl or ethyl and R is phenyl, 3-nitrophenyl, 3-halophenyl, or 3-chloro-5-bromophenyl;

(g) $R^1$ is phenyl or methyl and R is 3-methyl-4-halophenyl;

(h) X is oxygen, m is zero, R is 3,4-dichlorophenyl and $R^1$ is selected from phenyl, methyl and benzyl;

(i) m is zero, $R^1$ is phenyl, 4-nitrophenyl or 2,4-dinitrophenyl and R is methyl, phenyl, 4-tolyl, 4-chlorophenyl or 4-methoxyphenyl;

(j) m is zero, $R^1$ is phenyl, 2-nitrophenyl or ethyl and R is phenyl, 4-alkylphenyl, 4-halophenyl or 4-nitrophenyl;

(k) X is oxygen, m is zero, R is phenyl and $R^1$ is 4-alkylphenyl, 4-alkoxyphenyl or 4-halophenyl;

(l) X is oxygen, R is 4-halogen-3-methylphenyl and $R^1$ is methyl, phenyl or benzyl;

(m) X is oxygen, m is zero and $R^1$ is $C_{3-6}$ alkyl substituted by a chlorine atom;

(n) X is oxygen, m is zero, R is 4-chlorophenyl and $R^1$ is $-CH_2C{\equiv}CCH_2CH_3$;

(o) R is naphthyl, benzyl or methyl and $R^1$ is phenyl;

(p) R and $R^1$ are phenyl and m is greater than zero;

(q) R is dimethylphenyl and $R^1$ is phenyl, 2,5-dinitrophenyl, 4-methoxyphenyl, or $-CH_2$(N-piperidine);

(r) R is hydrogen or 4-ethylphenyl and $(R^2)_m$ is 6,7-dimethoxy;

(s) R is phenyl and $R^1$ is alkyl or-$(-CR^3R^4)_n$Het;

(t) R is 4-tolyl, $R^1$ is methyl or 4-methoxyphenyl and $(R^2)_m$ is tetrabromo;

(u) R is hydrogen and $R^1$ is phenyl, benzyl, or naphthyl;

(v) X is oxygen, R is hydrogen, $R^1$ is 2-tolyl, 2-fluorophenyl, 2-methoxyphenyl and $(R^2)_m$ is 7-ethylcarboxy or 7-carboxy;

(w) X is oxygen, R is hydrogen, $R^1$ is trifluoromethylphenyl and $(R^2)_m$ is 6-$NH_2$-7-Cl, 6-$SO_2NH_2$-7-Cl or 6-Cl-7-$SO_2NH_2$;

(x) R is hydrogen and $R^1$ is phenyl substituted in the 4-position by bromine, nitro or methoxy or in the 2-position by fluorine, methyl or methoxy;

(y) X is oxygen, R is hydrogen, $R^1$ is phenyl substituted in the 4-position by carboxy, nitro or fluorine, or in the 2-position by methyl, and $(R^2)_m$ is 8-carboxy or 8-methylcarboxy; and

(z) X is oxygen, R is hydrogen, $R^1$ is 4-nitrophenyl and $(R^2)_m$ is 7,8-dimethoxy.

In one embodiment, the invention provides a method of controlling the growth of weeds at a locus which comprises applying to the locus a herbicidally effective amount of a phthalazin-1-one or phthalazin-1-thione derivative of formula I as hereinbefore defined wherein:

R represents:-

a straight- or branched chain alkyl, alkenyl or alkynyl group containing from two to six carbon atoms optionally substituted by one or more halogen atoms;

a group $-(-CR^3R^4)_n$-(phenyl)-$(R^{5a})_q$;

a group $-(-CR^3R^4)_n$Het;

a group $-(-CR^3R^4)_n$-Ar, wherein Ar represents phenyl or pyridyl optionally substituted by one or more groups $R^{5a}$ and wherein two substituents on adjacent positions of the ring, together with the two atoms to

which they are attached, form a 5- to 7-membered alicyclic ring (which is optionally unsaturated) or an aromatic ring, optionally containing one or more heteroatoms (preferably selected from oxygen, sulphur and nitrogen, it being understood that a sulphur atom, where present, may be in the form of a group -SO- or $-SO_2-$), wherein the alicyclic or aromatic ring is optionally substituted by one or more groups $R^{51}$ which may be the same or different;

$R^1$ represents:-

a straight- or branched chain alkyl, alkenyl or alkynyl group containing from two to six carbon atoms optionally substituted by one or more halogen atoms;

a group $-(-CR^3R^4)_n$-(phenyl)-$(R^{5b})_q$;

a group $-(-CR^3R^4)_n$Het;

a group $-(-CR^3R^4)_n$-Ar, wherein Ar represents phenyl or pyridyl optionally substituted by one or more groups $R^{5b}$ and wherein two substituents on adjacent positions of the ring, together with the two atoms to which they are attached, form a 5- to 7-membered alicyclic ring (which is optionally unsaturated) or an aromatic ring, optionally containing one or more heteroatoms (preferably selected from oxygen, sulphur and nitrogen, it being understood that a sulphur atom, where present, may be in the form of a group -SO- or $-SO_2-$), wherein the alicyclic or aromatic ring is optionally substituted by one or more groups $R^{51}$ which may be the same or different;

provided that R represents a group $-(-CR^3R^4)_n$-(phenyl)-$(R^{5a})_q$ and/or $R^1$ represents a group $-(-CR^3R^4)_n$-(phenyl)-$(R^{5b})_q$ wherein n is zero;

and R is not phenyl mono-substituted in the 4-position by halogen;

$R^2$ represents:-

a group $R^5$;

or phenyl optionally substituted by from one to five groups $R^5$ which may be the same or different;

X represents oxygen or sulphur;

m represents zero or an integer from one to four;

where m is greater than one the groups $R^2$ may be the same or different;

$R^3$ and $R^4$, which may be the same or different, each represents the hydrogen atom or a straight- or branched- chain alkyl group containing up to four carbon atoms optionally substituted by one or more halogen atoms;

$R^5$, $R^{5a}$ and $R^{5b}$, which may be the same or different, each represents:-

a halogen atom;

a straight- or branched- chain alkyl, alkenyl or alkynyl group containing up to six carbon atoms optionally substituted by one or more halogen atoms; or

a group selected from cyano, nitro, $-CO_2R^6$, $-S(O)_pR^6$, $-NR^3R^4$, $-COR^6$, $-S(O)_pR^7$, $-CO_2R^7$, $-OR^7$, $-CONR^3R^4$, $-OSO_2R^7$, $-OSO_2R^8$, $-OCH_2R^7$, $-N(R^3)COR^8$, $-N(R^3)SO_2R^8$, $-N(R^3)SO_2R^7$, $-SO_2NR^3R^4$, $-Si(R^8)_3$ and $-OR^6$;

n represents zero, one or two; where n is two the groups $-(-CR^3R^4)-$ may be the same or different;

q represents zero or an integer from one to five; where q is greater than one the groups $R^5$ may be the same or different;

$R^{51}$ is as hereinbefore defined for $R^5$ or is =O or =S;

Het represents a 5- or 6- membered heterocycle containing from 3 to 5 carbon atoms in the ring and from 1 to 3 heteroatoms in the ring selected from nitrogen, sulphur and oxygen (e.g. pyridyl, pyrimidyl, thienyl or pyrazolyl) optionally substituted by one or more groups $R^5$ which may be the same or different;

$R^6$ represents the hydrogen atom or a straight- or branched-chain alkyl group containing up to six carbon atoms optionally substituted by one or more halogen atoms;

p represents zero, one or two;

$R^7$ represents phenyl optionally substituted by from one to five groups which may be the same or different selected from halogen, nitro, cyano, $R^6$ and $-OR^6$;

$R^8$ represents a straight- or branched- chain alkyl group containing up to six carbon atoms optionally substituted by one or more halogen atoms;

or an agriculturally acceptable salt thereof.

In this first embodiment the invention also provides a method using compounds of formula (I) in which m is zero, X is oxygen, R represents phenyl or 3-trifluoromethylphenyl and $R^1$ represents phenyl or 4-fluorophenyl.

In this first embodiment a number of compounds are novel and accordingly the present invention further provides compounds of formula (I) as hereinbefore defined in this first embodiment with the exclusion of the following compounds:

(a) X is oxygen, m is zero, $R^1$ is phenyl and R is $4-N(CH_3)_2$-phenyl;

(b) X is oxygen, R is alkyl and $(R^2)_m$ is 6,8-dimethyl-7-alkylcarboxy;

(c) X is oxygen, m is zero, $R^1$ is selected from the group consisting of methyl, phenyl, 4-chlorophenyl and 3-chlorophenyl and R is phenyl substituted in the 3,4 or 5- position by $-NH_2$ and optionally bearing one or two additional substituents selected from alkyl, halogen, alkoxy, hydroxy, phenoxy phenylsulphonamide and nitro;

(d) X is oxygen and $R^1$ is $-(CH_2)_n$Het wherein n is 2;

(e) $R^1$ is phenyl and R is $-(CR^3R^4)_n$Het;

(f) X is oxygen, m is zero, $R^1$ is 4-pyridylmethyl, benzyl or ethyl and R is phenyl, 3-nitrophenyl, 3-halophenyl, or 3-chloro-5-bromophenyl;

(g) $R^1$ is phenyl or methyl and R is 3-methyl-4-halophenyl;

(h) X is oxygen, m is zero, R is 3,4-dichlorophenyl and $R^1$ is selected from phenyl, methyl and benzyl;

(i) m is zero, $R^1$ is phenyl, 4-nitrophenyl or 2,4-dinitrophenyl and R is methyl, phenyl, 4-tolyl, or 4-methoxyphenyl;

(j) m is zero, $R^1$ is phenyl, 2-nitrophenyl or ethyl and R is phenyl, 4-alkylphenyl or 4-nitrophenyl;

(k) X is oxygen, m is zero, R is phenyl and $R^1$ is 4-alkylphenyl, 4-alkoxyphenyl or 4-halophenyl;

(l) X is oxygen, R is 4-halogen-3-methylphenyl and $R^1$ is methyl, phenyl or benzyl;

(m) X is oxygen, m is zero and $R^1$ is $C_{3-6}$ alkyl substituted by a chlorine atom;

(n) R is naphthyl, benzyl or methyl and $R^1$ is phenyl;

(o) R and $R^1$ are phenyl and m is greater than zero;

(p) R is dimethylphenyl and $R^1$ is phenyl, 2,5-dinitrophenyl, 4-methoxyphenyl, or $-CH_2$(N-piperidine);

(q) R is phenyl, $R^1$ is methyl and $(R^2)_m$ is 6,7-dimethoxy;

(r) R is phenyl and $R^1$ is alkyl or-$(-CR^3R^4)_n$Het; and

(s) R is 4-tolyl, $R^1$ is methyl or 4-methoxyphenyl and $(R^2)_m$ is tetrabromo.

A preferred class of compounds of formula I in this first embodiment because of their herbicidal properties are those having one or more of the following features:

R represents a group $-(-CR^3R^4-)_n-(\text{phenyl})-(R^{5a})_q$;

$R^1$ represents a group $-(-CR^3R^4-)_n-(\text{phenyl})-(R^{5b})_q$;

$R^2$ represents:-

a halogen atom;

a straight- or branched- chain alkyl, alkenyl or alkynyl group containing up to six carbon atoms optionally substituted by one or more halogen atoms; or

a group selected from cyano, nitro, $-CO_2R^6$, $-S(O)_pR^6$, $-NR^3R^4$, $-COR^6$, $-S(O)_pR^7$, $-CO_2R^7$, $-OR^7$, $-CONR^3R^4$, $-OSO_2R^7$ and $-OR^6$;

and X represents oxygen.

In this first embodiment preferably n is zero.

In this first embodiment, a further preferred class of compounds of formula I because of their herbicidal properties are those wherein:

R represents phenyl substituted in the 3-position by a group $R^{5a}$;

$R^1$ represents phenyl substituted in the 3- and/or 4- position by a group or groups $R^{5b}$;

$R^2$ represents:-

a halogen atom (e.g. fluorine or chlorine);

a straight- or branched- chain alkyl, alkenyl or alkynyl group containing up to six carbon atoms optionally substituted by one or more halogen atoms (e.g. methyl);

$R^{5a}$ represents:-

a halogen atom (preferably fluorine or chlorine);

a straight- or branched- chain alkyl or preferably alkoxy group containing up to four carbon atoms optionally substituted by one or more halogen atoms;

or a cyano group;

$R^{5b}$ represents:-

a halogen atom (preferably fluorine or chlorine);

a straight- or branched- chain alkyl group containing up to four carbon atoms optionally substituted by one or more halogen atoms;

or a cyano group;

m represents zero, one or two; and

X represents oxygen.

In this first embodiment another preferred class of compounds of formula (I) because of their herbicidal properties are those wherein:-

R represents methylenedioxyphenyl (preferably 2,3-methylenedioxyphenyl), wherein the methylene

group is optionally substituted by one or two (preferably two) groups $R^{51}$ which may be the same or different (preferably the same);

$R^1$ represents phenyl optionally substituted in the 3- and/or 4-position by $R^{5b}$;

$R^2$ represents:-

a halogen atom (preferably chlorine or fluorine);

a straight- or branched chain alkyl group containing up to four carbon atoms optionally substituted by one or more halogen atoms (e.g. methyl);

or a group selected from $-S(O)_pR^6$ and $-OR^6$ and, more preferably, $-NR^3R^4$;

$R^{5b}$ represents:-

a halogen atom (preferably fluorine or chlorine);

a straight- or branched chain alkyl group containing up to four carbon atoms optionally substituted by one or more halogen atoms;

or a cyano group;

m represents zero, one or two; and

X represents oxygen.

In this first embodiment preferably $R^{51}$ represents halogen, especially fluorine.

In this first embodiment, another particularly preferred class of compounds of formula (I) because of their herbicidal properties are those wherein:-

R represents:-

phenyl substituted in the 3-position by a group $R^{5a}$;

or 2,3-methylenedioxyphenyl wherein the methylene group is optionally substituted by one or preferably two groups $R^{51}$ which may be the same or different (preferably the same);

$R^1$ represents phenyl substituted in the 3- and/or 4-position by $R^{5b}$;

$R^2$ represents:-

a halogen atom, especially fluorine or chlorine;

a straight or branched chain alky group containing one to three carbon atoms, especially methyl;

$R^{5a}$ represents:-

an alkoxy group containing up to three carbon atoms substituted by from one to seven chlorine or fluorine atoms, (preferably trifluoromethoxy);

or 2,3-difluoromethylenedioxyphenyl;

$R^{5b}$ represents:-

a halogen atom (preferably fluorine);

a methyl group substituted by one to three halogens (preferably trifluoromethyl);

or a cyano group;

m represents zero, one or two (most preferably zero or one);

and X represents oxygen.

In this first embodiment where m is one or two preferably the groups $R^2$ are in the 6- and/or 7-position of the phthalazinone ring.

Where m is one and $R^2$ is methyl preferably $R^2$ occupies the 7- position of the phthalazinone ring.

In this first embodiment preferably R is phenyl monosubstituted in the 3-position by trifluoromethoxy.

The invention further provides herbicidal compositions comprising as active ingredient a herbicidally effective amount of a compound of formula (I) as defined in this first embodiment or an agriculturally acceptable salt thereof, in association with one or more compatible agriculturally- acceptable diluents or carriers.

In a second embodiment the invention provides a method for controlling the growth of pests at a locus which comprises applying to the locus a pesticidally effective amount of a phthalazin-1-one or phthalazin-1-thione derivative of formula I as hereinbefore defined wherein:

R represents:-

the hydrogen atom; or

phenyl optionally bearing from 1 to 5 substituents selected from the group consisting of halogen, alkyl, haloalkyl, cyano, alkoxy and haloalkoxy;

$R^1$ represents phenyl optionally bearing from 1 to 5 substituents selected from the group consisting of halogen, alkyl, haloalkyl, nitro, alkoxy, haloalkoxy and cyano;

$R^2$ represents:-

a member of the group consisting of halogen, alkyl, haloalkyl, nitro, alkoxy, haloalkoxy and cyano;

X represents oxygen or sulphur; and

m is zero or an integer from 1 to 4;

or an agriculturally acceptable salt thereof.

In this second embodiment a number of these compounds are novel and accordingly the invention further provides compounds of formula (I) as herebefore defined in this second embodiment with the exclusion of the following compounds:

(a) X is oxygen, m is zero, $R^1$ is phenyl and R is 4-$N(CH_3)_2$-phenyl;

(b) $R^1$ is phenyl and R is 3-methyl-4-halophenyl;

(c) X is oxygen, m is zero, R is 3,4-dichlorophenyl and $R^1$ is phenyl;

(d) m is zero, $R^1$ is phenyl, 4-nitrophenyl or 2,4-dinitrophenyl and R is phenyl, 4-tolyl, 4-chlorophenyl or 4-methoxyphenyl;

(e) m is zero, $R^1$ is phenyl or 2-nitrophenyl and R is phenyl, 4-alkylphenyl or 4-halophenyl;

(f) X is oxygen, m is zero, R is phenyl and $R^1$ is 4-alkylphenyl, 4-alkoxyphenyl or 4-halophenyl;

(g) X is oxygen, R is 4-halogen-3-methylphenyl and $R^1$ is phenyl;

(h) R and $R^1$ are phenyl and m is greater than zero;

(i) R is dimethylphenyl and $R^1$ is phenyl, 2,5-dinitrophenyl or 4-methoxyphenyl;

(j) R is hydrogen or phenyl and $(R^2)_m$ is 6,7-dimethoxy;

(k) R is 4-tolyl, $R^1$ is 4-methoxyphenyl and $(R^2)_m$ is tetrabromo;

(l) R is hydrogen and $R^1$ is phenyl;

(m) R is hydrogen and $R^1$ is phenyl substituted in the 4-position by bromine, nitro or methoxy or in the 2-position by fluorine, methyl or methoxy; and

(n) X is oxygen, R is hydrogen, $R^1$ is 4-nitrophenyl and $(R^2)_m$ is 7,8-dimethoxy.

In this second embodiment, preferably R represents phenyl optionally monosubstituted in the 4-position.

In this second embodiment compounds wherein R is phenyl substituted by 1 to 3 (most preferably 1) halogen atoms are also preferred.

Also preferred in this second embodiment are compounds of formula I wherein $R^1$ is phenyl substituted by one or two substituents selected from the group consisting of nitro, halogen haloalkyl (e.g. $CF_3$) and haloalkoxy (e.g. $-OCF_3$).

In this second embodiment compounds of formula I wherein $R^2$ is in the 6- or 7-position of the phthalazinone ring (especially the 7- position) are also preferred.

In this second embodiment a further preferred class of compounds of formula I are those wherein $R^2$ is halogen (preferably chlorine or fluorine).

Preferably X represents oxygen.

In this second embodiment compounds of formula I wherein m is zero, one or two are also preferred.

In this second embodiment another preferred class of compounds of formula I because of their insecticidal properties are those wherein:

R represents hydrogen; or

phenyl bearing halogen;

$R^1$ represents phenyl bearing one or two members of the group consisting of nitro, alkyl, and haloalkoxy;

$R^2$ represents halogen; and

m is zero one or two.

In this second embodiment another class of compounds of formula I are those wherein R represents hydrogen or phenyl monosubstituted in the 4-position by halogen.

The invention further provides pesticidal compositions comprising as active ingredient a pesticidally effective amount of a compound of formula (I) as defined in this second embodiment or an agriculturally acceptable salt thereof,in association with, and preferably homogeneously dispersed in, one or more compatible agriculturally- acceptable diluents or carriers.

Particularly preferred compounds of formula I include the following:

1. 2-(2,4-difluorophenyl)-4-(3-trifluoromethylphenyl)phthalazin-1-one,
2. 2-(4-fluorophenyl)-4-(3-trifluoromethoxyphenyl)phthalazin-1-one,
3. 4-(3-trifluoromethoxyphenyl)-2-(4-trifluoromethylphenyl)phthalazin-1-one,
4. 4-(2,3-difluoromethylenedioxyphenyl)-2-(4-fluorophenyl)phthalazin-1-one,
5. 4-(4-bromophenyl)-2-(2,4-dinitrophenyl)phthalazin-1-one,
6. 2-(4-trifluoromethoxyphenyl)phthalazin-1-one,
7. 2-(4-trifluoromethylphenyl)phthalazin-1-one,
8. 2-(4-chlorophenyl)-4-(4-fluorophenyl)phthalazin-1-one,
9. 4-(4-fluorophenyl)-2-(4-trifluoromethyoxyphenyl)phthalazin-1-one,
10. 2-(4-chlorophenyl)-7-fluoro-4-(4-fluorophenyl)phthalazin-1-one,
11. 7-fluoro-4-(4-fluorophenyl)-2-(4-trifluoromethoxyphenyl)phthalazin-1-one,

12. 8-fluoro-2(4-fluorophenyl)-4-(3-trifluoromethoxyphenyl)phthalazin-1-one,
13. 2-(4-fluorophenyl)-7-methyl-4-(3-trifluoromethoxyphenyl)phthalazin-1-one,
14. 4-(2,3-difluoromethylenedioxyphenyl)-2-(4-trifluoromethylphenyl)phthalazine-1-one,
15. 2-(4-chlorophenyl)-4-(2,3-difluoromethylenedioxyphenyl)-phthalazin-1-one,
16. 2-(4-chlorophenyl)-4-(3-trifluoromethylphenyl)phthalazin-1-one,
17. 7-chloro-2-(4-fluorophenyl)-4-(3-trifluoromethoxyphenyl)phthalazin-1-one,
18. 2-(4-fluorophenyl)-7-methyl-4-(3-trifluoromethoxyphenyl)phthalazin-1-one,
19. 2-(4-chlorophenyl)-4-(3-trifluoromethoxyphenyl)phthalazin-1-one,
20. 4-(2,3-difluoromethylenedioxyphenyl)-2-(4-fluorophenyl)-7-methylphthalazin-1-one,
21. 7-chloro-4-(2,3-difluoromethylenedioxyphenyl)-2-(4-fluorophenyl)phthalazin-1-one,
22. 4-(2,3-difluoromethylenedioxyphenyl)-7-methyl-2-(4-trifluoromethylphenyl)phthalazin-1-one,
23. 4-(2,3-difluoromethylenedioxyphenyl)-2-(5-trifluoromethylpyrid-2-yl)phthalazin-1-one,
24. 7-methyl-4-(3-trifluoromethoxyphenyl)-2-(4-trifluoromethylphenyl)phthalazin-1-one,
25. 7-fluoro-2-(4-fluorophenyl)-4-(3-trifluoromethylphenyl)phthalazin-1-one,
26. 2-(4-fluorophenyl)-7-methoxy-4-(3-trifluoromethoxyphenyl)phthalazin-1-one,
27. 6-fluoro-2-(4-fluorophenyl)-4-(3-trifluoromethoxyphenyl)phthalazin-1-one,
28. 7-methoxy-4-(3-trifluoromethoxyphenyl)-2-(4-trifluoromethylphenyl)phthalazin-1-one,
29. 7-chloro-4-(3-trifluoromethoxyphenyl)-2-(4-trifluoromethylphenyl)phthalazin-1-one,
30. 2-(4-fluorophenyl)-4-(3-trifluoromethoxyphenyl)-7-trifluoromethoxyphthalazin-1-one,
31. 2-(4-fluorophenyl)-4-(3-trifluoromethoxyphenyl)-7-trifluoromethylphthalazin-1-one,
32. 7-fluoro-4-(3-trifluoromethoxyphenyl)-2-(4-fluorophenyl)phthalazin-1-one,
33. 7-chloro-4-(2,3-difluoromethylenedioxyphenyl)-2-(4-trifluoromethylphenyl)phthalazin-1-one,
34. 2-(4-fluorophenyl)phthalazin-1-one,
35. 4-(2,3-difluoromethylenedioxyphenyl)-7-fluoro-2-(4-fluorophenyl)phthalazin-1-one,
36. 4-(2,3-difluoromethylenedioxyphenyl)-7-fluoro-2-(4-trifluoromethylphenyl)phthalazin-1-one,
37. 5-fluoro-2-(4-fluorophenyl)-4-(3-trifluoromethoxyphenyl)phthalazin-1-one,
38. 2-(4-fluorophenyl)-4-(pyrid-3-yl)phthalazin-1-one,
39. 4-(3-ethylphenyl)-2-(4-fluorophenyl)phthalazin-1-one,
40. 2-(4-fluorophenyl)-7-nitro-4-(3-trifluoromethoxyphenyl)phthalazin-1-one,
41. 2-(4-fluorophenyl)-6-nitro-4-(3-trifluoromethoxyphenyl)phthalazin-1-one,
42. 6-amino-2-(4-fluorophenyl)-4-(3-trifluoromethoxyphenyl)phthalazin-1-one,
43. 7-amino-2-(4-fluorophenyl)-4-(3-trifluoromethoxyphenyl)phthalazin-1-one,
44. 2-(4-fluorophenyl)-4-(3-trifluoromethylphenyl)phthalazin-1-one,
45. 2,4-diphenylphthalazin-1-one,
46. 2-phenyl-4-(3-trifluoromethylphenyl)phthalazin-1-one,
47. 2-(2,4,6-trichlorophenyl)phthalazin-1-one,
48. 2-(4-chlorophenyl)phthalazin-1-one,
49. 6,7-dichloro-4-(4-chlorophenyl)-2(4-trifluoromethoxyphenyl)phthalazin-1-one,
50. 6,7-dichloro-2-(4-chlorophenyl)-4-(4-fluorophenyl)phthalazin-1-one,
51. 6,7-dichloro-4-(4-fluorophenyl)-2-(4-trifluoromethoxyphenyl)phthalazin-1-one, and
52. 2-(4-fluorophenyl)-4-(4-fluorophenyl)phthalazin-1-one.

The numbers 1 to 52 are assigned to these compounds for reference and identification hereafter.

Compound numbers 1 to 4,12 to 33 and 35 to 46 are particularly useful for their herbicidal properties.

Compound numbers 5 to 11,34 and 47 to 52 are particularly useful for their ability to control a number of pest species.

Compounds of formula I may be prepared by the application or adaptation of known methods (i.e. methods heretofore used or described in the literature), for example as hereinafter described.

In the following description, where symbols appearing in formulae are not specifically defined, it is to be understood that they are "as hereinbefore defined" in accordance with the first definition of each symbol in the specification.

It is to be understood that in the description of the following processes the sequences may be performed in different orders, and that suitable protecting groups may be required to achieve the compounds sought.

According to a feature of the present invention compounds of formula I wherein X represents oxygen may be prepared by the reaction of a compound of formula II:

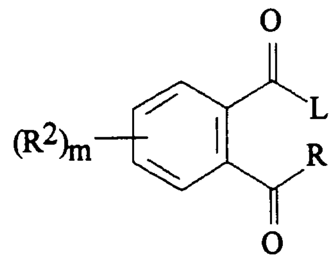

(II)

wherein R, $R^2$ and m are as hereinbefore defined, with a hydrazine of formula III or a salt thereof:

$R^1$-$NHNH_2$ (III)

wherein $R^1$ is as hereinbefore defined and L is a leaving group. Generally L is -OH, straight- or branched- chain alkoxy containing up to 4 carbon atoms (e.g. ethoxy), or halogen, for example chlorine. The reaction is generally carried out in a solvent such as toluene or ethanol. Where the hydrazine of formula III is used in the form of a salt (such as the hydrochloride) the reaction is generally performed in the presence of a base or acid acceptor such as triethylamine, sodium acetate or potassium carbonate. The reaction is generally performed from room temperature to the reflux temperature of the mixture and preferably with azeotropic removal of water from the mixture.

According to a further feature of the present invention compounds of formula (I) in which X represents oxygen may be prepared by the cyclisation of a compound of formula (IIa):

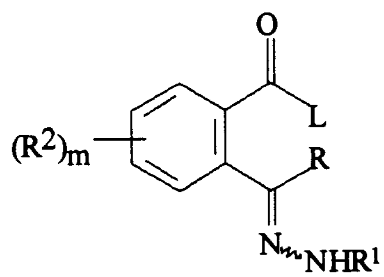

(IIa)

wherein R, $R^1$, $R^2$, m and L are as hereinbefore defined. The reaction is generally carried out in an alcoholic solvent such as methanol or ethanol in the presence of a base or acid acceptor such as triethylamine, sodium acetate or potassium carbonate optionally in the presence of a catalyst, for example para-toluenesulphonic acid. The reaction is generally performed at a temperature from room temperature to the reflux temperature of the mixture and preferably with azeotropic removal of water from the mixture.

A number of the compounds of formula IIa are novel and as such constitute a further feature of the present invention.

According to a further feature of the present invention, compounds of formula (I) wherein X represents oxygen and R represents $-(-CR^3R^4)_n-(phenyl)-(R^{5a})_q$, $-(-CR^3R^4-)_nHet$ or $-(-CR^3R^4-)_n-Ar$, may be prepared by the reaction of a compound of formula IV:

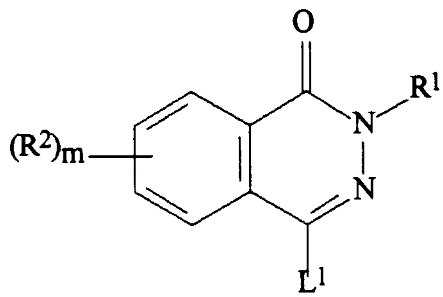

(IV)

wherein $R^1$, $R^2$ and m are as hereinbefore defined and $L^1$ is halogen, for example chlorine, bromine or iodine with a Grignard reagent of formula V;

$R\text{-}MgL^1$ (V)

wherein $L^1$ is as hereinbefore defined and R represents alkyl, $-(-CR^3R^4)_n$-(phenyl)-$(R^{5a})_q$, $-(-CR^3R^4-)_n$Het or $-(CR^3R^4-)_n$-Ar. The reaction is performed in the presence of a nickel catalyst, for example 1,3-bis-(diphenylphosphino)propane nickel (II) chloride in a suitable solvent, for example tetrahydrofuran at a temperature from room temperature to the reflux temperature of the mixture. This type of reaction is well documented in the literature (for example, as described by Tamao et al., Tetrahedron, 1982, 38, 3347).

In a modification of the reaction described above, compounds of formula I wherein R represents alkenyl or alkynyl may be prepared by the reaction of a compound of formula IV wherein $R^1$, $R^2$, m and $L^1$ are as hereinbefore defined, with the appropriate alkene or alkyne in the present of a catalyst, for example palladium diacetate or palladium dichloride. The reactions are widely described in the literature (for example, by Heck et al, J.O.C., 1978, 43, 2967 and by Sonogashira et al, Tett. Lett., 1975, 4467).

According to a further feature of the invention compounds of formula I wherein X is oxygen and R is hydrogen may be prepared by the reaction of a compound of formula Va:

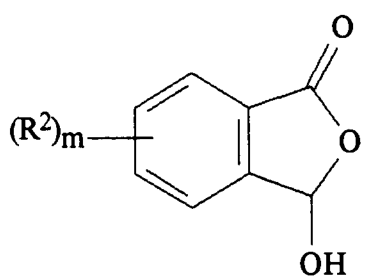

(Va)

wherein $R^2$ and m are as hereinbefore defined with an appropriately substituted hydrazine of formula III wherein R is as hereinbefore defined, or a salt thereof.

The reaction is carried out in a solvent such as ethanol, acetic acid or water. Where the hydrazine of formula III is used in the form of a salt (such as the hydrochloride) the reaction is generally performed in the presence of a base or acid acceptor such as triethylamine, sodium acetate or potassium carbonate. The reaction is generally performed at room temperature to the reflux temperature of the mixture and preferably with azeotropic removal of water from the mixture.

According to a further feature of the invention compounds of formula I where R is alkyl, $-(-CR^3R^4)_n$-(phenyl)-$(R^{5a})_q$, $-(-CR^3R^4-)_n$Het or $-(-CR^3R^4-)_n$-Ar, wherein n is one or two, and X is oxygen may be prepared by the reaction of a compound of formula VI:

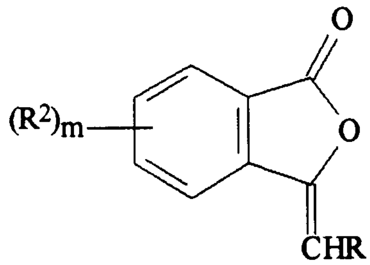

(VI)

wherein $R^2$ and m are as hereinbefore defined and R represents alkyl, $-(-CR^3R^4)_n-(phenyl)-(R^{5a})_q$, $-(-CR^3R^4)_nHet$ or $-(-CR^3R^4-)_n-Ar$, wherein n is one or two, with a hydrazine of formula III wherein $R^1$ is as hereinbefore defined or a salt thereof. The reaction is carried out in a solvent such as ethanol, acetic acid or water. Where the hydrazine of formula III is used in the form of a salt (such as the hydrochloride) the reaction is generally performed in the presence of a base or acid acceptor such as triethylamine, sodium acetate or potassium carbonate. The reaction is generally performed at room temperature to the reflux temperature of the mixture and preferably with azeotropic removal of water from the mixture.

According to a further feature of the invention compounds of formula I in which X represents oxygen and $R^1$ represents alkyl, alkenyl, alkynyl or $-(-CR^3R^4)_n-(phenyl)-(R^{5b})_q$, $-(-CR^3R^4-)_nHet$ or $-(-CR^3R^4-)_n-Ar$, wherein n is one or two, may be prepared by the reaction of the corresponding compound of formula I in which $R^1$ is replaced by hydrogen with a compound of formula VII:

$R^1-L^2$ (VII)

wherein $R^1$ represents alkyl, alkenyl, alkynyl or a group selected from $-(-CR^3R^4)_n-(phenyl)-(R^{5b})_q$, $-(-CR^3R^4-)_nHet$ and $-(-CR^3R^4-)_n-Ar$, wherein n is one or two, and $L^2$ is a leaving group such as halogen. Where $R^1$ represents alkenyl or alkynyl, $L^2$ is not attached to an unsaturated carbon atom. The reaction is carried out in a suitable solvent with an appropriate base for example sodium hydroxide in water or sodium methoxide in dimethylsulphoxide. The reaction is performed at a temperature from room temperature to the reflux temperature of the mixture.

According to a further feature of the present invention compounds of formula I wherein X represents sulphur may be prepared from corresponding compounds of formula I in which X represents oxygen by reaction with a thionation reagent to convert the carbonyl group to a thiocarbonyl group. Suitable thionation reagents include Lawessons' Reagent, i.e. [2,4-bis(4-methoxyphenyl)-1,3-dithia-2,4-diphosphetane-2,4-disulphide], and phosphorus pentasulphide. The reaction is generally carried out in a suitable solvent, for example toluene, at a temperature from 50°C to the reflux temperature of the mixture.

Intermediates in the preparation of compounds of formula I may be prepared by application or adaptation of known methods.

Compounds of formula II wherein L is OH or alkoxy containing up to 4 carbon atoms may be prepared by the oxidation of a compound of formula VIII:

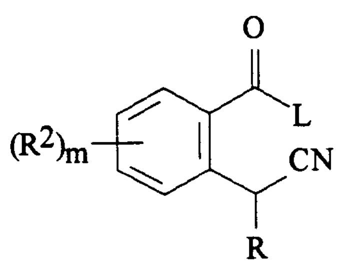

(VIII)

wherein R, $R^2$ and m are as hereinbefore defined, to convert the cyanomethylene group to a carbonyl group. The reaction is carried out in the presence of a base, for example lithium diisopropylamide,

potassium carbonate or sodium hydride, in an anhydrous solvent, for example dimethylsulphoxide, 1,4-dioxane or tetrahydrofuran at temperatures from -72°C to the reflux temperature of the mixture. Generally the oxidant used is air or oxygen. Alternatively, the reaction may be carried out in a twophase system comprising an organic solvent such as toluene or dichloromethane and an aqueous solution of a base, for example sodium hydroxide, in the presence of a quaternary ammonium salt, for example triethyl benzylammonium chloride. Generally the oxidant used is air or oxygen. The reaction is generally performed at a temperature from room temperature to the reflux temperature of the mixture.

Compounds of formula II wherein L is alkoxy containing up to 4 carbon atoms or halogen may be prepared from the corresponding carboxylic acid of formula II in which L represents OH by the application or modification of known methods.

Compounds of formula II wherein L is OH may be prepared by the reaction of an appropriately substituted phthalic anhydride of formula IX:

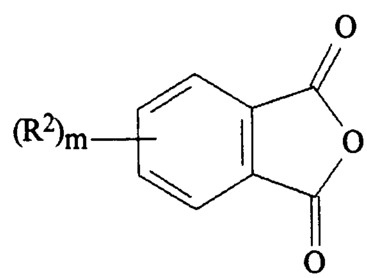

(IX)

wherein $R^2$ and m are as hereinbefore defined, with an organometallic reagent of formula R-M, wherein R is as hereinbefore defined and M represents magnesium (as provided in Grignard reagents, for example RMgCl), or lithium (provided as R-Li, typically generated by reacting a compound of formula R-Br with butyl lithium). The reaction is generally carried out in a solvent such as diethyl ether, tetrahydrofuran or toluene, or a mixture containing combinations of these solvents) at a temperature from -70°C to the reflux temperature of the mixture.

Compounds of formula II wherein R represents $-[-CR^3R^4-]_n-(phenyl)-(R^{5a})_q$, n is zero and L is OH may be prepared by a Friedel Crafts reaction between a phthalic anhydride of formula IX as hereinbefore defined and a compound of formula $-(phenyl)-(R^{5a})_q$. The reaction is generally performed in a solvent such as 1,2-dichloroethane or 1,1,2,2-tetrachloroethane, in the presence of a Lewis acid catalyst, preferably aluminium trichloride. The reaction is typically performed at a temperature from room temperature to the reflux temperature of the mixture.

Compounds of formula (II) in which L represents hydroxy may be prepared by the oxidation of a toluene derivative of formula (IXa):

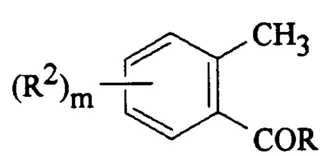

(IXa)

to oxidise the methyl group to a caboxylic acid. The oxidation is typically carried out using an oxidant such as chromium (VI) oxide or potassium permangate, in a solvent such as sulphuric acid, acetic acid or pyridine.

Compounds of formula (II) in which L represents hydroxy may also be prepared by the reaction of carboxylic acid derivative of formula (IXb)

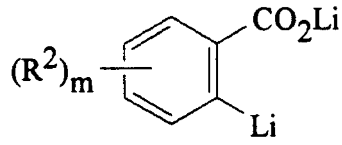

(IXb)

wherein $R^2$ and m are as hereinbefore defined, with a compound of formula $RCOL^3$ wherein $L^3$ is a leaving group such as chlorine or alkoxy. The reaction is generally carried out at a temperature from -100°C to reflux temperature in a solvent such as tetrahydrofuran. The compound of formula (IXb) is typically formed in situ from the corresponding carboxylic acid by treatment with at least 2 moles of a lithium reagent (such as an alkyl lithium or lithium diisopropylamide).

Compounds of formula IIa may be prepared by the reaction of a compound of formula II with a hydrazine of formula III or a salt thereof. The reaction is generally performed in a solvent such as ethanol or methanol optionally in the presence of a base, for example triethylamine or potassium carbonate (the presence of a base is particularly preferred where a salt of formula III is used) and a catalyst, for example para-toluene sulphonic acid. The reaction is generally carried out at a temperature from ambient to the reflux temperature of the mixture.

Compounds of formula IV may be prepared by halogenation of a compound of formula X:

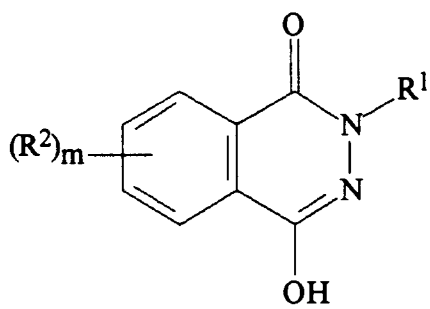

(X)

wherein $R^1$, $R^2$ and m are as hereinbefore defined. The reaction is carried under conditions widely described in the literature (using, for example, phosphorus trichloride, phosphorus tribromide or hydrogen iodide).

Compounds of formula VI may be prepared by the condensation of a phthalic anhydride of formula IX wherein $R^2$ and m are as hereinbefore defined, with an acetic acid derivative of formula $R\text{-}CH_2CO_2H$, wherein R represents alkyl, $-(-CR^3R^4)_n-(phenyl)-(R^{5a})_q$, $-(-CR^3R^4-)_nHet$ or $-(-CR^3R^4-)_n-Ar$ and n is one or two The reaction is described in the literature (for example, Islam et al, Chemical Abstracts, 1978, 88, 170075n).

Compounds of formula I in which $R^1$ is replaced by hydrogen, X represents oxygen and $R^2$ and m are as hereinbefore defined may be prepared by the reaction of a compound of formula II with hydrazine or a salt thereof as described above for the reaction of a compound of formula II with a compound of formula III.

Compounds of formula VIII in which L is alkoxy containing up to 4 carbon atoms, or preferably OH, may be prepared by the reaction of a benzoic acid derivative of formula (XI):

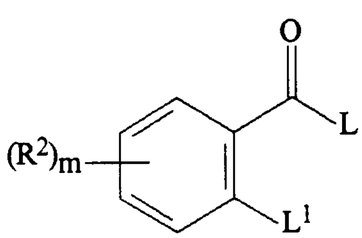

(XI)

wherein $R^2$, m and $L^1$ are as hereinbefore defined and L is alkoxy containing up to 4 carbon atoms or OH, and a nitrile of formula $RCH_2CN$ wherein R is as hereinbefore defined. The reaction is performed in the presence of a base, preferably sodium amide, in a solvent, preferably liquid ammonia, at a temperature from -33°C. In a modification to the above reaction, where L is OH and $L^1$ represents bromine or iodine, the condensation may be performed in the presence of a copper halide catalyst ( preferably cuprous bromide) and a base (preferably sodium hydride) in a solvent, e.g. toluene. The reaction is generally performed from 0°C to the reflux temperature of the mixture.

Compounds of formula VIII in which R represents straight- or branched- chain alkyl, alkenyl or alkynyl containing up to six carbon atoms optionally substituted by one or more halogen atoms, or a group selected from $-[-CR^3R^4]_n-(phenyl)-(R^{5a})_q$, $-[CR^3R^4]_n-(Het)$ and $-[-CR^3R^4]_n-Ar$ wherein n is one or two, and L is -OH or alkoxy containing up to 4 carbon atoms, may be prepared by the reaction of a 2-cyanomethylbenzoic acid derivative of formula XII:

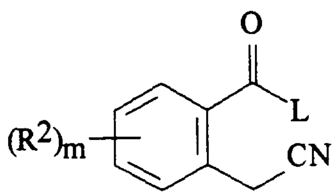

(XII)

wherein $R^2$ and m are as hereinbefore defined and L is -OH or alkoxy containing up to 4 carbon atoms, with a compound of formula $R-L^2$ , wherein R represents straight- or branched- chain alkyl, alkenyl or alkynyl containing up to six carbon atoms optionally substituted by one or more halogen atoms, or a group selected from $-[CR^3R^4]_n-(phenyl)-(R^{5a})_q$, $-[CR^3R^4]_n-(Het)$ and $-[-CR^3R^4]_n-Ar$ wherein n is one or two and $L^2$ is as defined above. The reaction is performed in the presence of a base and is widely described in the literature (e.g. Masuyama et al, Chem. Lett., 1977, 1439).

Compounds of formula (IXa) may be prepared by the reaction of a toluene derivative of formula (XIIa):

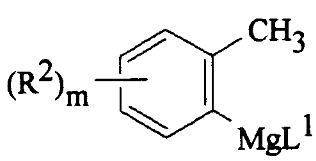

(XIIa)

wherein $R^2$, m and $L^1$ are as hereinbefore defined, with a compound of formula $RCOL^3$ wherein $L^3$ is as hereinbefore defined. Preferably $L^1$ is bromine. The reaction is generally carried out in a solvent (e.g. tetrahydrofuran, ether or toluene) at a temperature from -70°C to the reflux temperature.

Compounds of formula X may be prepared by the reaction of either a phthalic anhydride derivative of formula IX as hereinbefore defined or a phthalic acid derivative of formula XIII:

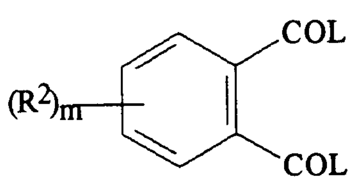

(XIII)

wherein $R^2$, m and L are as hereinbefore defined, with a hydrazine of formula III or a salt thereof. Generally L is -OH, alkoxy containing up to 4 carbon atoms (e.g. ethoxy) or halogen, for example chlorine. The reaction is generally performed as described above for the reaction between compounds of formulae II and III.

Compounds of formula XII wherein L is OH or alkoxy may be prepared by the cyanation of a compound of formula XIV:

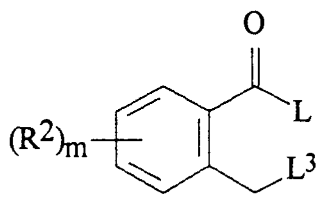

(XIV)

wherein $R^2$ and m are as hereinbefore defined, L is -OH or alkoxy and $L^3$ is a leaving group (e.g. chlorine or bromine). The cyanide source is, for example, sodium cyanide. The reaction is conducted in a solvent, for example aqueous ethanol at a temperature between room temperature and the reflux temperature of the mixture.

Compounds of formula XIV in which $L^3$ is halogen may be prepared by the halogenation of a toluene derivative for formula XV:

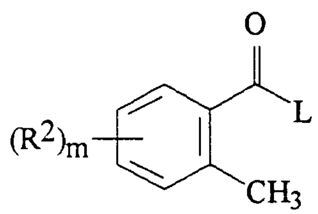

(XV)

wherein $R^2$, m and L are as hereinbefore defined. The reaction is performed in the presence of suitable halogen source, for example N-bromo- or N-chlorosuccinimide, or chlorine or bromine, in a solvent, for example carbon tetrachloride or chloroform, at a temperature between room temperature and the reflux temperature of the mixture. The reaction is preferably conducted in the presence of a reaction initiator, for example benzoyl peroxide.

Compounds of formulae III, V, Va, VII, IX, IXb, XI, XIIa, XIII and XV are known or may be prepared by the application or modification of known methods.

The following Examples illustrate the preparation of compounds of the formula I and the Reference Examples illustrate the preparation of intermediates. In the present specification m.p. means melting point; NMR means nuclear magnetic resonance spectrum. Unless otherwise stated percentages are by weight.

## Example 1

A mixture of 2-(3-trifluoromethylbenzoyl)benzoic acid (2g), 2,4-difluorophenylhydrazine hydrochloride (1.47 g) and triethylamine (0.83g) in toluene was heated at reflux for 19 hours with azeotropic removal of water (Dean-Stark apparatus). The reaction mixture was cooled to room temperature and filtered. The filtrate was washed successively with 0.5N hydrochloric acid, aqueous sodium carbonate, water and brine. The organic phase was dried ($MgSO_4$), filtered and evaporated. The residue was purified by column chromatography to yield a yellow solid which was recrystallised from isopropyl ether/hexane to give 2-(2,4-difluorophenyl)-4-(3-trifluoromethylphenyl)phthalazin-1-one, compound 1, as pale tan crystals (0.9 g), m.p. 102-103.5°C.

By proceeding in a similar manner, the following compounds of formula I may be prepared:

2. 2-(4-fluorophenyl)-4-(3-trifluoromethoxyphenyl)phthalazin-1-one;
4. 4-(2,3-difluoromethylenedioxyphenyl)-2-(4-fluorophenyl)phthalazine-1-one, m.p. 142-144.6°C;
5. 4-(4-bromophenyl)-2-(2,4-dinitrophenyl)phthalazin-1-one, m.p. 98-101°C;
6. 2-(4-trifluoromethoxyphenyl)phthalazin-1-one, m.p. 113 -115.5°C;
7. 2-(4-trifluoromethylphenyl)phthalazin-1-one, m.p. 158-159°C;

8. 2-(4-chlorophenyl)-4-(4-fluorophenyl)phthalazin-1-one,
9. 4-(4-fluorophenyl)-2-(4-trifluoromethyoxyphenyl)phthalzin-1-one, m.p. 121-129°C;
10. 2-(4-chlorophenyl)-7-fluoro-4-(4-fluorophenyl)phthalazin-1-one, m.p. 202.5-204.5°C;
11. 7-fluoro-4-(4-fluorophenyl)-2-(4-trifluoromethoxyphenyl)phthalazin-1-one, m.p. 119-121°C;
47. 2-(2,4,6-trichlorophenyl)phthalazin-1-one, m.p. 195-198.5°C;
48. 2-(4-chlorophenyl)phthalazin-1-one, m.p. 162-164°C;
49. 6,7-dichloro-4-(4-chlorophenyl)-2-(4-trifluoromethoxyphenyl)phthalazin-1-one, m.p. 179-181°C;
50. 6,7-dichloro-2-(4-chlorophenyl)-4-(4-fluorophenyl)phthalazin-1-one, m.p. 212-214°C;
51. 6,7-dichloro-4-(4-fluorophenyl)-2-(4-trifluoromethoxyphenyl)phthalazin-1-one, m.p. 159-160°C and
52. (4-fluorophenyl)-4-(4-fluorophenyl)phthalazin-1-one, m.p. 154-155°C.

## Example 2

2-(3-trifluoromethoxybenzoyl)benzoic acid (3.26g) was added to a suspension of 4-fluorophenyl hydrazine hydrochloride (2.23g) in ethanol, followed by the addition of sodium acetate (1.12g). The reaction mixture was stirred at the reflux temperature of the mixture for 7 hours. The solvent was evaporated and the residue partitioned between water and dichloromethane. After separation, the aqueous phase was extracted with dichloromethane and the combined extracts washed with 2M hydrochloric acid followed by water. The dichloromethane solution was dried ($MgSO_4$), filtered and the dichloromethane evaporated to give an orange oil. This was passed through a short column of silica to yield an orange solid which was triturated with cyclohexane to yield 2-(4-fluorophenyl)-4-(3-trifluoromethoxyphenyl)phthalazin-1-one (compound 2, 2.53g) as a fawn solid, m.p. 109-111°C.

By proceeding in a similar manner the following compounds of formula I were prepared:-

12. 8-fluoro-2-(4-fluorophenyl)-4-(3-trifluoromethoxyphenyl)phthalazin-1-one, m.p. 128-129°C;
13. 2-(4-fluorophenyl)-7-methyl-4-(3-trifluoromethoxyphenyl)phthalazin-1-one, m.p. 106-109°C;
14. 4-(2,3-difluoromethylenedioxyphenyl)-2-(4-trifluoromethylphenyl)phthalazine-1-one, m.p. 176-178°C;
15. 2-(4-chlorophenyl)-4-(2,3-difluoromethylenedioxyphenyl)phthalazin-1-one, m.p. 143.2-145°C;
16. 2-(4-chlorophenyl)-4-(3-trifluoromethylphenyl)phthalazin-1-one, m.p. 154.6-155.8°C;
17. 7-chloro-2-(4-fluorophenyl)-4-(3-trifluoromethoxyphenyl)phthalazin-1-one, m.p. 142.3-142.5°C (cyclisation carried out in toluene, base triethylamine);
18. 2-(4-fluorophenyl)-7-methyl-4-(3-trifluoromethoxyphenyl)phthalazin-1-one, m.p. 106-109°C (cyclisation carried out in ethanol, base sodium acetate);
19. 2-(4-chlorophenyl)-4-(3-trifluoromethoxyphenyl)phthalazin-1-one, m.p. 105-106°C;
20. 4-(2,3-difluoromethylenedioxyphenyl)-2-(4-fluorophenyl)-7-methylphthalazin-1-one, m.p. 166-168°C;
21. 7-chloro-4-(2,3-difluoromethylenedioxyphenyl)-2-(4-fluorophenyl)phthalazin-1-one, m.p. 163-164°C;
22. 4-(2,3-difluoromethylenedioxyphenyl)-7-methyl-2-(4-trifluoromethylphenyl)phthalazin-1-one, m.p. 187-189°C;
23. 4-(2,3-difluoromethylenedioxyphenyl)-2-(5-trifluoromethylpyrid-2-yl)phthalazin-1-one, m.p. 112-112.8°C;
24. 7-methyl-4-(3-trifluoromethoxyphenyl)-2-(4-trifluoromethylphenyl)phthalazin-1-one, m.p. 137-138°C;
25. 7-fluoro-2-(4-fluorophenyl)-4-(3-trifluoromethylphenyl)phthalazin-1-one, m.p. 135-138°C;
26. 2-(4-fluorophenyl)-7-methoxy-4-(3-trifluoromethoxyphenyl)phthalazin-1-one, m.p. 135.1-137.5°C;
27. 6-fluoro-2-(4-fluorophenyl)-4-(3-trifluoromethoxyphenyl)phthalazin-1-one, m.p. 76-78°C;
28. 7-methoxy-4-(3-trifluoromethoxyphenyl)-2-(4-trifluoromethylphenyl)phthalazin-1-one, m.p. 121.8-122.2°C;
29. 7-chloro-4-(3-trifluoromethoxyphenyl)-2-(4-trifluoromethylphenyl)phthalazin-1-one, m.p. 113-114°C;
30. 2-(4-fluorophenyl)-4-(3-trifluoromethoxyphenyl)-7-trifluoromethoxyphthalazin-1-one, m.p. 121.5-123.5°C;
31. 2-(4-fluorophenyl)-4-(3-trifluoromethoxyphenyl)-7-trifluoromethylphthalazin-1-one, m.p. 102.4-103.3°C;
32. 7-fluoro-4-(3-trifluoromethoxyphenyl)-2-(4-fluorophenyl)phthalazin-1-one, m.p. 106.6-107.1°C;
33. 7-chloro-4-(2,3-difluoromethylenedioxyphenyl)-2-(4-trifluoromethylphenyl)phthalazin-1-one, m.p. 182-184°C;
34. 2-(4-fluorophenyl)phthalazin-1-one, m.p. 134-136°C;
35. 4-(2,3-difluoromethylenedioxyphenyl)-7-fluoro-2-(4-fluorophenyl)phthalazin-1-one, m.p. 132.2-133.4°C;
36. 4-(2,3-difluoromethylenedioxyphenyl)-7-fluoro-2-(4-trifluoromethylphenyl)phthalazin-1-one, m.p. 165.9-167.8°C;
37. 5-fluoro-2-(4-fluorophenyl)-4-(3-trifluoromethoxyphenyl)phthalazin-1-one, m.p. 66-70°C;
38. 2-(4-fluorophenyl)-4-(pyrid-3-yl)phthalazin-1-one, m.p. 196-197°C;

39. 4-(3-ethylphenyl)-2-(4-fluorophenyl)phthalazin-1-one, m.p.118-119°C;
40. 2-(4-fluorophenyl)-7-nitro-4-(3-trifluoromethoxyphenyl)phthalazin-1-one, m.p. 143.9-145°C; and
41. 2-(4-fluorophenyl)-6-nitro-4-(3-trifluoromethoxyphenyl)phthalazin-1-one, m.p. 106.8-108.6°C.

**Example 3**

A solution of 2-(3-trifluoromethoxybenzoyl)benzoic acid (0.76g) and 4-trifluoromethylphenylhydrazine (0.88g) in toluene was refluxed for 18 hours with azeotropic removal of water (by a Dean-Stark apparatus). The reaction was diluted with diethyl ether and washed successively with 2N hydrochloric acid, brine, saturated sodium bicarbonate solution and brine. The organic phase was dried ($MgSO_4$), filtered and evaporated. The residue was dissolved in dichloromethane and passed through a short column of silica. The eluent was evaporated and the crude product was recrystallised from cyclohexane/hexane to yield 4-(3-trifluoromethoxyphenyl)-2-(4-trifluoromethylphenyl)-phthalazin-1-one (compound 3, 0.36g) as a yellow solid, m.p. 113.2-116°C.

**Example 4**

A solution of 2-(4-fluorophenyl)-6/7-nitro-4-(3-trifluoromethoxyphenyl)phthalazin-1-one (1.6g) in ethanol was added to a solution of tin (II) chloride (1.9g) in concentrated hydrochloric acid and ethanol at ambient temperature. After stirring for 7 hours, the solvent was evaporated and the residue partitioned between ethyl acetate and 2N sodium hydroxide. The aqueous phase was extracted with ethyl acetate and the combined organic extracts dried ($MgSO_4$). The suspension was filtered and the solvent evaporated. The residue was passed through a short column of silica to yield 6-amino-2-(4-fluorophenyl)-4-(3-trifluoromethoxyphenyl)-phthalazin-1-one (compound 42, 0.41g) as a cream solid, m.p. 157.8-159.2°C and 7-amino-2-(4-fluorophenyl)-4-(3-trifluoromethoxyphenyl)phthalazin-1-one (compound 43, 0.34g) as a cream solid, m.p. 148.9-151.3°C.

**Example 5**

2-(3-Trifluoromethylbenzoyl)benzoic acid (2g) was stirred in toluene and 4-fluorophenylhydrazine hydrochloride (1.33 g) was added followed by triethylamine (0.83g). The mixture was heated at reflux temperature for 21 hours with azeotropic removal of water (Dean-Stark apparatus). The reaction mixture was cooled to room temperature and filtered. The filtrate was washed successively with 0.5N hydrochloric acid, aqueous sodium carbonate and water. The organic phase was dried ($MgSO_4$), filtered and evaporated. The residue was recrystallised from n-butanol to give 2-(4-fluorophenyl)-4-(3-trifluoromethylphenyl)phthalazin-1-one, compound 44, 1.73g as fawn crystals, m.p. 146.5 - 147°C.

By proceeding in a similar manner, the following compounds of formula I were prepared:

45. 2,4-diphenylphthalazin-1-one, m.p. 166 - 168°C
46. 2-phenyl-4-(3-trifluoromethylphenyl)phthalazin-1-one, m.p. 138 - 140°C.

**Reference Example 1**

3-Bromobenzotrifluoride (11.29g) in ether was added to a stirred suspension of magnesium turnings (1.46g) in dry ether at reflux temperature. The resulting Grignard reagent was stirred at 20 to 30°C for a further 1 hour, then added to a stirred suspension of phthalic anhydride (7.3g) in toluene/ether at 0°C. The suspension was then stirred for 4 hours at 90°C and left to stand overnight. The mixture was poured into a mixture of ice and 2N hydrochloric acid solution, the organic phase separated and the aqueous phase extracted with ether. The combined organic extracts were dried (magnesium sulphate), the solvent evaporated, the residue was taken up into dichloromethane and extracted with sodium carbonate solution. The combined aqueous extracts were filtered cooled and acidified (concentrated hydrochloric acid). The resulting precipitate was collected by filtration and dried to yield 2-(3-trifluoromethylbenzoyl)benzoic acid as a beige solid (9.02g), m.p. 164-166.5°C.

By proceeding in a similar manner the following compounds were prepared:

2-(3-trifluoromethoxybenzoyl)-benzoic acid, NMR ($CDCl_3$): 7.32-7.77 (7H,m), 8.1(1H,d).
2-(3-ethylbenzoyl)benzoic acid, m.p. 88°-94°C;
4-nitro-2(3-trifluoromethoxybenzoyl)benzoic acid and
5-nitro-2-(3-trifluoromethoxybenzoyl)benzoic acid, obtained as a mixture.

## Reference Example 2

A solution of n-butyl lithium (2.5M in hexane; 8.8ml) was added to a stirred, cooled (-78°C) solution of 1,2-difluoromethylenedioxybenzene (3.16g) in dry tetrahydrofuran under an inert atmosphere. The mixture was stirred at -78°C for a further 3 hours and was then transferred to a jacketed dropping funnel (with solid carbon dioxide as coolant). This anion solution was added to a stirred, cooled (-78°C) suspension of phthalic anhydride (2.96 g) in dry tetrahydrofuran so that the temperature of the reaction mixture did not exceed -65°C. The resulting suspension was stirred at -70°C for a further 30 minutes then allowed to warm to 0°C. The mixture was then recooled to -70°C and 2N hydrochloric acid was added. The solution was then poured into water and the mixture extracted with ethyl acetate. The organic phase was washed with 2N hydrochloric acid then brine, dried (magnesium sulphate) and evaporated. The residue was purified by column chromatography using dichloromethane/ethyl acetate to yield 2-(2,3-difluoromethylenedioxybenzoyl)benzoic acid as a white solid (1.56g), m.p. 131.2-132.2°C.

## Reference Example 3

2-bromo-4-fluorotoluene (10.5g) in dry tetrahydrofuran was added to a suspension of magnesium turnings (1.44g) in dry tetrahydrofuran at reflux temperature. The resulting Grignard reagent was stirred at reflux for a further 1 hour, then allowed to cool to ambient temperature before adding to a solution of 3-trifluoromethoxybenzoyl chloride (10.0g) in dry tetrahydrofuran at 0°C. The reaction mixture was stirred for a further 30 minutes and then saturated ammonium chloride solution was added to precipitate an oily solid. This was extracted with ethyl acetate and then washed with water. The ethyl acetate solution was then dried ($MgSO_4$), filtered and the solvent evaporated to give 5-fluoro-2-methyl-3'-trifluoromethoxybenzophenone 4-fluoro-2-(3-trifluoromethoxybenzoyl)benzoic acid(13.65g) as an orange oil, NMR ($CDCl_3$): 2.3(3H,s), 7.05-(1H,d of d), 7.15(1H,m), 7.3(1H,m), 7.5(2H,m), 7.7(2H,m).

## Reference Example 4

5-fluoro-2-methyl-3'-trifluoromethoxybenzophenone (13.0g) was dissolved in glacial acetic acid (60ml) and a solution of chromium (VI) oxide (11.6g) in 30% sulphuric acid and acetic acid was added with stirring. The reaction mixture was heated to reflux for 6 hours. A solution of chromium (VI) oxide (4.36g) in 30% sulphuric acid and acetic acid was added and refluxing was continued overnight. A further molar equivalent of chromium (VI) oxide (4.36g) in 30% sulphuric acid and acetic acid was added and was heated at reflux for a further 7 hours. The reaction mixture was allowed to stand at ambient temperature overnight and was then poured into water. The aqueous suspension was extracted with ethyl acetate. The combined organic extracts were washed with water, dried ($MgSO_4$), filtered and the solvent evaporated to give a green oil which was triturated with hexane, ether to yield 4-fluoro-2-(3-trifluoromethoxybenzoyl)benzoic acid (3.2g) as a green solid, m.p. 92-95°C.

## Reference Example 5

60% Sodium hydride (0.86g) was suspended in dry dimethylformamide and 3-trifluoromethoxyphenylacetonitrile (2.17g) was added with stirring under an inert atmosphere. After 15 minutes, ethyl 2-chloro-5-trifluoromethylbenzoate (2.47g) was added dropwise, causing the temperature to rise to 40°C. After 5 minutes, the reaction mixture was heated to 50°C and air was bubbled through the suspension for 2 hours.

The mixture was poured onto ice, water and acidified with 2N hydrochloric acid and extracted with ether. The combined organic extracts were washed with water, dried ($MgSO_4$) and the solvent evaporated to give the ester as a brown oil (3.8g), which was dissolved in ethanol. Water and lithium hydroxide (1.17g) was added with stirring. After 1 hour, the majority of the ethanol was evaporated and the residue poured into water. The aqueous solution was washed with hexane, acidified to pH 2 with concentrated hydrochloric acid and extracted with ethyl acetate. The combined organic extracts were dried ($MgSO_4$), the solvent evaporated and the residue triturated with cyclohexane/ether to yield 2-(3-trifluoromethoxybenzoyl)-5-trifluoromethylbenzoic acid, (2.25g) as a buff solid, m.p. 127.6-130.5°C.

**Reference Example 6**

2.5 M Butyl lithium in hexane (18.5ml) was added under an inert atmosphere to a solution of 3-fluorobenzoic acid (3.0g) in dry tetrahydrofuran at -75°C. The reaction mixture was stirred at -78°C overnight. A solution of methyl 3-trifluoromethoxybenzoate (4.6g) in dry tetrahydrofuran was added and after 2 hours the reaction mixture was allowed to warm to -20°C. 2N hydrochloric acid was added, the mixture and the organic layer was separated and dried ($MgSO_4$). Evaporated the solvent and triturated the residue with hexane to yield 3-fluoro-2-(3-trifluoromethoxybenzoyl)benzoic acid (4.3g) as a white solid, m.p. 135-140°C.

**Reference Example 7**

2-Bromo-6-fluorobenzoic acid (2.0g) was dissolved in dry tetrahydrofuran and 2.5 M butyl lithium (8.0ml) in hexane was added under an inert atmosphere at -75°C. After stirring for one hour, methyl 3-trifluoromethoxybenzoate (2.0g) was added in dry tetrahydrofuran over 20 minutes. Alter stirring at -75°C for a further 2 hours the reaction mixture was allowed to warm to ambient temperature. Water was added to the reaction mixture which was then acidified to pH 2 with 2N hydrochloric acid. The layers were separated and the aqueous phase was extracted with ethyl acetate. The combined extracts were washed with water, dried ($MgSO_4$) and evaporated. The residue was passed through a short column of silica to yield 6-fluoro-2-(3-trifluoromethoxybenzoyl)benzoic acid (1.1g) as a buff solid, m.p. 210-216°C.

**Reference Example 8**

To a suspension of 2-bromo-5-chlorobenzoic acid (1.2g) in toluene (35ml) was added 3-trifluoromethoxyphenylacetonitrile (1.4g), followed by 60% sodium hydride (0.6g) and copper (I) bromide (0.09g). The reaction mixture was heated to reflux, under an inert atmosphere for 3.5 hours. After cooling to 70°C air was bubbled through the reaction mixture for 1 hour. The mixture was poured into water, the organic phase separated and the aqueous layer was washed with ether. The aqueous layer was then acidified to pH 2 with 2N hydrochloric acid and extracted with ethyl acetate. The combined ethyl acetate extracts were washed with brine, dried ($MgSO_4$) and evaporated to yield 5-chloro-2-(3-trifluoromethoxybenzoyl)benzoic acid as a pale yellow solid (1.27g), NMR (DMSO $d_6$) 7.6(2H,m), 7.65(2H,m), 7.8(2H,m), 7.9(1H,m), 13.7(1H,broad s).

By proceeding in a similar manner, the following compounds were prepared:-

5-methyl-2-(3-trifluoromethoxybenzoyl)benzoic acid, m.p. 78-82°C;

2-(2,3-difluoromethylenedioxybenzoyl)-5-methylbenzoic acid, NMR ($CDCl_3$) 2.5(3H,s), 7.1(2H,m), 7.3-(2H,m), 7.5(1H,m), 7.95(1H,m);

5-chloro-2-(2,3-difluoromethylenedioxybenzoyl)benzoic acid, NMR ($CDCl_3$) $^{19}F$ 50.0;

5-fluoro-2-(3-trifluoromethoxybenzoyl)benzoic acid, NMR (DMSO $d_6$) 7.4(2H,m), 7.6(2H,m), 7.65(1H,m), 7.75(2H,m);

5-methoxy-2-(3-trifluoromethoxybenzoyl)benzoic acid;

5-trifluoromethoxy-2-(3-trifluoromethoxybenzoyl)benzoic acid, NMR ($CDCl_3$) 7.5(5H,m), 7.9(1H,d), 8.0-(1H, dd).

According to a further feature of the present invention, there are provided compositions suitable for herbicidal or insecticidal use comprising one or more of the phthalazin-1-one and phthalazin-1-thione derivatives of formula I or an agriculturally acceptable salt thereof,in association with, and preferably homogeneously dispersed in, one or more compatible agriculturally- acceptable diluents or carriers and/or surface active agents [i.e. diluents or carriers and/or surface active agents of the type generally accepted in the art as being suitable for use in herbicidal or insecticidal compositions and which are compatible with compounds of formula I.

The term "homogeneously dispersed" is used to include compositions in which the compounds of formula I are dissolved in other components. The terms "herbicidal compositions" and "insecticidal compositions" are used in a broad sense to include not only compositions which are ready for use as herbicides or insecticides but also concentrates which must be diluted before use. Preferably, the compositions contain from 0.05 to 90% by weight of one or more compounds of formula I.

The herbicidal or insecticidal compositions may contain both a diluent or carrier and surface-active (e.g. wetting, dispersing, or emulsifying) agent. Surface-active agents which may be present in herbicidal or insecticidal compositions of the present invention may be of the ionic or non-ionic types, for example sulphoricinoleates, quaternary ammonium derivatives, products based on condensates of ethylene oxide with alkyl and polyaryl phenols, e.g. nonyl- or octyl-phenols, or carboxylic acid esters of anhydrosorbitols

which have been rendered soluble by etherification of the free hydroxy groups by condensation with ethylene oxide, alkali and alkaline earth metal salts of sulphuric acid esters and sulphonic acids such as dinonyl- and dioctyl-sodium sulphonosuccinates and alkali and alkaline earth metal salts of high molecular weight sulphonic acid derivatives such as sodium and calcium lignosulphonates and sodium and calcium alkylbenzene sulphonates.

Suitably, the herbicidal or insecticidal compositions according to the present invention may comprise up to 10% by weight, e.g. from 0.05% to 10% by weight, of surface-active agent but, if desired, herbicidal or insecticidal compositions according to the present invention may comprise higher proportions of surface-active agent, for example up to 15% by weight in liquid emulsifiable suspension concentrates and up to 25% by weight in liquid water soluble concentrates.

Examples of suitable solid diluents or carriers are aluminium silicate, microfine silicon dioxide, talc, chalk, calcined magnesia, kieselguhr, tricalcium phosphate, powdered cork, adsorbent carbon black and clays such as kaolin and bentonite. The solid compositions (which may take the form of dusts, granules or wettable powders) are preferably prepared by grinding the compounds of formula I with solid diluents or by impregnating the solid diluents or carriers with solutions of the compounds of formula I in volatile solvents, evaporating the solvents and, if necessary, grinding the products so as to obtain powders. Granular formulations may be prepared by absorbing the compounds of formula I (dissolved in suitable solvents, which may, if desired, be volatile) onto the solid diluents or carriers in granular form and, if desired, evaporating the solvents, or by granulating compositions in powder form obtained as described above. Solid herbicidal or insecticidal compositions, particularly wettable powders and granules, may contain wetting or dispersing agents (for example of the types described above), which may also, when solid, serve as diluents or carriers.

Liquid compositions according to the invention may take the form of aqueous, organic or aqueous-organic solutions, suspensions and emulsions which may incorporate a surface-active agent. Suitable liquid diluents for incorporation in the liquid compositions include water, glycols, glycol ethers, tetrahydrofurfuryl alcohol, acetophenone, cyclohexanone, isophorone, N-alkyl pyrrolidones, toluene, xylene, mineral, animal and vegetable oils, esterified vegetable oils and light aromatic and naphthenic fractions of petroleum (and mixtures of these diluents). Surface-active agents, which may be present in the liquid compositions, may be ionic or non-ionic (for example of the types described above) and may, when liquid, also serve as diluents or carriers.

Powders, dispersible granules and liquid compositions in the form of concentrates may be diluted with water or other suitable diluents, for example mineral or vegetable oils, particularly in the case of liquid concentrates in which the diluent or carrier is an oil, to give compositions ready for use.

When desired, liquid compositions of the compound of formula I may be used in the form of self-emulsifying concentrates containing the active substances dissolved in the emulsifying agents or in solvents containing emulsifying agents compatible with the active substances, the simple addition of such concentrates to water producing compositions ready for use.

Liquid concentrates in which the diluent or carrier is an oil may be used without further dilution using the electrostatic spray technique.

Herbicidal or insecticidal compositions according to the present invention may also contain, if desired, conventional adjuvants such as adhesives, protective colloids, thickeners, penetrating agents, spreading agents, stabilisers, sequestering agents, anti-caking agents, colouring agents and corrosion inhibitors. These adjuvants may also serve as carriers or diluents.

Unless otherwise specified, the following percentages are by weight. Preferred herbicidal or insecticidal compositions according to the present invention are

aqueous suspension concentrates which comprise from 10 to 70% of one or more compounds of formula I, from 2 to 10% of surface-active agent, from 0.1 to 5% of thickener and from 15 to 87.9% of water;

wettable powders which comprise from 10 to 90% of one or more compounds of formula I, from 2 to 10% of surface-active agent and from 8 to 88% of solid diluent or carrier;

water soluble or water dispersible powders which comprise from 10 to 90% of one or more compounds of formula I, from 2 to 40% of sodium carbonate and from 0 to 88% of solid diluent;

liquid water soluble concentrates which comprise from 5 to 50%, e.g. 10 to 30%, of one or more compounds of formula I, from 0 to 25% of surface-active agent and from 10 to 90%, e.g. 45 to 85%, of water miscible solvent, e.g. triethylene glycol, or a mixture of water-miscible solvent and water;

liquid emulsifiable suspension concentrates which comprise from 10 to 70% of one or more compounds of formula I, from 5 to 15% of surface-active agent, from 0.1 to 5% of thickener and from 10 to 84.9% of organic solvent, e.g. mineral oil;

water dispersible granules which comprise from 1 to 90%, e.g. 25 to 75% of one or more compounds of formula I, from 1 to 15%, e.g. 2 to 10%, of surface-active agent and from 5 to 95%, e.g. 20 to 60%, of solid diluent, e.g. clay, granulated with the addition of water to form a paste and then dried and

emulsifiable concentrates which comprise 0.05 to 90%, and preferably from 1 to 60% of one or more compounds of formula I, from 0.01 to 10%, and preferably from 1 to 10%, of surface-active agent and from 9.99 to 99.94%, and preferably from 39 to 98.99%, of organic solvent.

Herbicidal or insecticidal compositions according to the present invention may also comprise the compounds of formula I in association with, and preferably homogeneously dispersed in, one or more other pesticidally active compounds and, if desired, one or more compatible pesticidally acceptable diluents or carriers, surface-active agents and conventional adjuvants as hereinbefore described.

Examples of other pesticidally active compounds which may be included in, or used in conjunction with, the herbicidal or insecticidal compositions of the present invention include herbicides, for example to increase, where applicable, the range of weed species controlled for example alachlor [2-chloro-2,6'-diethyl-N-(methoxy-methyl)-acetanilide], atrazine [2-chloro-4-ethylamino-6-isopropylamino-1,3,5-triazine], bromoxynil [3,5-dibromo-4-hydroxybenzonitrile], chlortoluron [N'-(3-chloro-4-methylphenyl)-N,N-dimethylurea], cyanazine [2-chloro-4-(1-cyano-1-methylethylamino)-6-ethylamino-1,3,5-triazine], 2,4-D [2,4-dichlorophenoxy-acetic acid], dicamba [3,6-dichloro-2-methoxybenzoic acid], difenzoquat [1,2- dimethyl-3,5-diphenylpyrazolium salts], flampropmethyl [methyl N-2-(N- benzoyl-3-chloro-4-fluoroanilino)-propionate], fluometuron [N'-(3-trifluoromethylphenyl)-N,N-dimethylurea], isoproturon [N'-(4-isopropylphenyl)-N,N-dimethylurea], insecticides for example acephate, chlorpyrifos, demeton-S-methyl, disulfoton, ethoprofos, fenitrothion, fenamiphos, fonofos, isazophos, isofenphos, malathion, monocrotophos, parathion, phorate, phosalone, pirimiphos-methyl, terbufos, triazophos, cyfluthrin, cypermethrin, deltamethrin, fenpropathrin, fenvalerate, permethrin, tefluthrin, aldicarb, carbosulfan, methomyl, oxamyl, pirimicarb, bendiocarb, teflubenzuron, dicofol, endosulfan, lindane, benzoximate, cartap, cyhexatin, tetradifon, avermectins, ivermectins, milbemycins, thiophanate, trichlorfon, dichlorvos, diaveridine or dimetriadazole,

and fungicides, e.g. carbamates, e.g. methyl N-(1-butylcarbamoyl- benzimidazol-2-yl)carbamate, and triazoles e.g. 1-(4-chloro-phenoxy)-3,3- dimethyl-1-(1,2,4-triazol-1-yl)-butan-2-one.

Pesticidally active compounds and other biologically active materials which may be included in, or used in conjunction with, the herbicidal or insecticidal compositions of the present invention, for example those hereinbefore mentioned, and which are acids, may, if desired, be utilized in the form of conventional derivatives, for example alkali metal and amine salts and esters.

The following Examples illustrate pesticidal compositions according to the present invention:

## EXAMPLE C1

A wettable powder is formed from:

| | |
|---|---|
| Active ingredient (compound 1) | 80% w/w |
| Sodium dodecylbenzene sulphonate | 3% w/w |
| Sodium N-methyl-N-oleyl taurate | 2% w/w |
| Sodium polycarboxylate | 1% w/w |
| Microfine silicon dioxide | 2% w/w |
| China clay | 12% w/w |

by blending the above ingredients and grinding the mixture in an air jet mill.

Similar wettable powders may be prepared as described above by replacing the phthalazin-1-one derivative (compound 1) with other compounds of formula I.

**EXAMPLE C2**

A suspension concentrate is formed from:

| | |
|---|---|
| Active ingredient (compound 1) | 60% w/v |
| Nonyl phenol 9 mole polyethoxylate | 0.5% w/v |
| Triethanolamine salt of phosphated tristyryl phenol 16 mole polyethoxylate | 1.5% w/v |
| Sodium polycarboxylate | 0.4% w/v |
| polysaccharide gum | 0.1% w/v |
| propylene glycol | 5% w/v |
| silicone antifoam emulsion | 0.01% w/v |
| 1,2-benzisothiazolin-3-one solution in dipropylene glycol | 0.01% w/v |
| water | to 100 volumes |

by mixing using a high shear mixer all ingredients into 90% volume of water, then making up to volume with water, then milling the mixture by passing through a horizontal bead mill.

Similar suspension concentrates may be prepared as described above by replacing the phthalazin-1-one derivative (compound 1) with other compounds of formula I.

**EXAMPLE C3**

A granule is formed from

| | |
|---|---|
| Active ingredient (compound 1) | 5% w/w |
| Sepiolite granules 30/60 mesh | 95% w/w |

by dissolving the active ingredient in n-butanol, then spraying this solution onto sepiolite granules whilst mixing the granules in a tumbler-mixer then evaporating off the n-butanol to leave a granule containing 5% w/w active ingredient.

Similar granules may be prepared as described above by replacing the phthalazin-1-one derivative (compound 1) with other compounds of formula I.

**EXAMPLE C4**

A water dispersible granule is formed from

| | |
|---|---|
| Active ingredient (compound 1) | 75% w/w |
| Sodium lignosulphonate | 10% w/w |
| Sodium dialkylnaphthalene sulphonate | 3% w/w |
| Clay | 12% w/w |

by blending the above ingredients, then grinding the mixture in an air-jet mill, then adding water to form a kneadable paste, then extruding this paste to form fine filaments approximately 1mm in diameter, chopping the extrudate into lengths of approximately 4mm then drying these in a fluid bed drier.

Similar water dispersible granules may be prepared as described above by replacing the phthalazin-1-one (compound 1) with other compounds of formula I.

According to a feature of the present invention, there is provided a method for controlling the growth of weeds (i.e. undesired vegetation) at a locus which comprises applying to the locus a herbicidally effective amount of at least one phthalazin-1-one and phthalazin-1-thione derivative of formula (I) or an agriculturally acceptable salt thereof. For this purpose, the phthalazin-1-one and phthalazin-1-thione derivatives are normally used in the form of herbicidal compositions (i.e. in association with compatible diluents or carriers and/or surface active agents suitable for use in herbicidal compositions), for example as hereinafter described.

The compounds of formula (I) show herbicidal activity against dicotyledonous (i.e. broad-leafed) and monocotyledonous (e.g. grass) weeds by pre- and/or post-emergence application.

By the term "pre-emergence application" is meant application to the soil in which the weed seeds or seedlings are present before emergence of the weeds above the surface of the soil. By the term "post-emergence application" is meant application to the aerial or exposed portions of the weeds which have emerged above the surface of the soil. For example, the compounds of formula (I) may be used to control the growth of:

broad-leafed weeds, for example, Abutilon theophrasti, Amaranthus retroflexus, Bidens pilosa, Chenopodium album, Galium aparine, Ipomoea spp. e.g. Ipomoea purpurea, Sesbania exaltata, Sinapis arvensis, Solanum nigrum and Xanthium strumarium, and

grass weeds, for example Alopecurus myosuroides, Avena fatua, Digitaria sanguinalis, Echinochloa crus-galli, Eleusine indica and Setaria spp, e.g. Setaria faberii or Setaria viridis, and

sedges, for example, Cyperus esculentus.

The amounts of compounds of formula (I) applied vary with the nature of the weeds, the compositions used, the time of application, the climatic and edaphic conditions and (when used to control the growth of weeds in crop-growing areas) the nature of the crops. When applied to a crop-growing area, the rate of application should be sufficient to control the growth of weeds without causing substantial permanent damage to the crop. In general, taking these factors into account, application rates between 0.01kg and 5kg of active material per hectare give good results. However, it is to be understood that higher or lower application rates may be used, depending upon the particular problem of weed control encountered.

The compounds of formula (I) may be used to control selectively the growth of weeds, for example to control the growth of those species hereinbefore mentioned, by pre- or post-emergence application in a directional or non-directional fashion, e.g. by directional or non-directional spraying, to a locus of weed infestation which is an area used, or to be used, for growing crops, for example cereals, e.g. wheat, barley, oats, maize and rice, soya beans, field and dwarf beans, peas, lucerne, cotton, peanuts, flax, onions, carrots, cabbage, oilseed rape, sunflower, sugar beet, and permanent or sown grassland before or after sowing of the crop or before or after emergence of the crop. For the selective control of weeds at a locus of weed infestation which is an area used, or to be used, for growing of crops, e.g. the crops hereinbefore mentioned, application rates between 0.01kg and 4.0kg, and preferably between 0.01kg and 2.0kg, of active material per hectare are particularly suitable.

The compounds of formula (I) may also be used to control the growth of weeds, especially those indicated above, by pre- or post-emergence application in established orchards and other tree-growing areas, for example forests, woods and parks, and plantations, e.g. sugar cane, oil palm and rubber plantations. For this purpose they may be applied in a directional or non- directional fashion (e.g. by directional or non-directional spraying) to the weeds or to the soil in which they are expected to appear, before or after planting of the trees or plantations at application rates between 0.25kg and 5.0kg, and preferably between 0.5kg and 4.0kg of active material per hectare.

The compounds of formula (I) may also be used to control the growth of weeds, especially those indicated above, at loci which are not crop-growing areas but in which the control of weeds is nevertheless desirable.

Examples of such non-crop-growing areas include airfields, industrial sites, railways, roadside verges, the verges of rivers, irrigation and other waterways, scrublands and fallow or uncultivated land, in particular where it is desired to control the growth of weeds in order to reduce fire risks. When used for such purposes in which a total herbicidal effect is frequently desired, the active compounds are normally applied at dosage rates higher than those used in crop-growing areas as hereinbefore described. The precise dosage will depend upon the nature of the vegetation treated and the effect sought.

Pre- or post-emergence application, and preferably pre-emergence application, in a directional or non-directional fashion (e.g. by directional or non-directional spraying) at application rates between 1.0kg and 20.0kg, and preferably between 5.0 and 10.0kg, of active material per hectare are particularly suitable for this purpose.

When used to control the growth of weeds by pre-emergence application, the compounds of formula (I) may be incorporated into the soil in which the weeds are expected to emerge. It will be appreciated that when the compounds of formula (I) are used to control the growth of weeds by post-emergence application, i.e. by application to the aerial or exposed portions of emerged weeds, the compounds of formula (I) will also normally come into contact with the soil and may also then exercise a pre-emergence control on later-germinating weeds in the soil.

Where especially prolonged weed control is required, the application of the compounds of formula (I) may be repeated if required.

Representative compounds of formula (I) have been used in herbicidal applications according to the following procedures.

**METHOD OF USE OF HERBICIDAL COMPOUNDS:**

a) General

Appropriate quantities of the compounds used to treat the plants were dissolved in acetone to give solutions equivalent to application rates of up to 4000g test compound per hectare (g/ha). These solutions were applied from a standard laboratory herbicide sprayer delivering the equivalent of 290 litres of spray fluid per hectare.

b) Weed control : Pre-emergence

The seeds were sown in 70 mm square, 75 mm deep plastic pots in non-sterile soil. The quantities of seed per pot were as follows:-

| Weed species | Approx number of seeds/pot |
|---|---|
| 1) Broad-leafed weeds | |
| Abutilon theophrasti | 10 |
| Amaranthus retroflexus | 20 |
| Galium aparine | 10 |
| Ipomoea purpurea | 10 |
| Sinapis arvensis | 15 |
| Xanthium strumarium | 2. |
| 2) Grass weeds | |
| Alopecurus myosuroides | 15 |
| Avena fatua | 10 |
| Echinochloa crus-galli | 15 |
| Setaria viridis | 20. |
| 3) Sedges | |
| Cyperus esculentus | 3. |
| Crop | |
| 1) Broad-leafed | |
| Cotton | 3 |
| Soya | 3. |
| 2) Grass | |
| Maize | 2 |
| Rice | 6 |
| Wheat | 6. |

The compounds of the invention were applied to the soil surface, containing the seeds, as described in (a). A single pot of each crop and each weed was allocated to each treatment, with unsprayed controls and controls sprayed with acetone alone.

After treatment the pots were placed on capillary matting kept in a glass house, and watered overhead. Visual assessment of crop damage was made 20-24 days after spraying. The results were expressed as the percentage reduction in growth or damage to the crop or weeds, in comparison with the plants in the control pots.

c) Weed control: Post-emergence

The weeds and crops were sown directly into John Innes potting compost in 75 mm deep, 70 mm square pots except for Amaranthus which was pricked out at the seedling stage and transferred to the pots one week before spraying. The plants were then grown in the greenhouse until ready for spraying with the

compounds used to treat the plants. The number of plants per pot were as follows:-

| 1) Broad leafed weeds | | |
|---|---|---|
| Weed species | Number of plants per pot | Growth stage |
| Abutilon theophrasti | 3 | 1-2 leaves |
| Amaranthus retroflexus | 4 | 1-2 leaves |
| Galium aparine | 3 | $1^{st}$ whorl |
| Ipomoea purpurea | 3 | 1-2 leaves |
| Sinapis arvensis | 4 | 2 leaves |
| Xanthium strumarium | 1 | 2-3 leaves. |

| 2) Grass weeds | | |
|---|---|---|
| Weed species | Number of plants per pot | Growth stage |
| Alopecurus myosuroides | 8-12 | 1-2 leaves |
| Avena fatua | 12-18 | 1-2 leaves |
| Echinochloa crus-galli | 4 | 2-3 leaves |
| Setaria viridis | 15-25 | 1-2 leaves. |

| 3) Sedges | | |
|---|---|---|
| Weed species | Number of plants per pot | Growth stage |
| Cyperus esculentus | 3 | 3 leaves. |

| 1) Broad leafed | | |
|---|---|---|
| Crops | Number of plants per pot | Growth stage |
| Cotton | 2 | 1 leaf |
| Soya | 2 | 2 leaves. |

| 2) Grass | | |
|---|---|---|
| Crops | Number of plants per pot | Growth stage |
| Maize | 2 | 2-3 leaves |
| Rice | 4 | 2-3 leaves |
| Wheat | 5 | 2-3 leaves. |

The compounds used to treat the plants were applied to the plants as described in (a). A single pot of each crop and weed species was allocated to each treatment, with unsprayed controls and controls sprayed with acetone alone.

After treatment the pots were placed on capillary matting in a glass house, and watered overhead once after 24 hours and then by controlled sub-irrigation. Visual assessment of crop damage and weed control was made 20-24 days after spraying. The results were expressed as the percentage reduction in growth or damage to the crop or weeds, in comparison with the plants in the control pots.

When applied at 4000g/hectare or less pre- or post-emergence, compounds 1 to 4,12 to 33 and 35 to 46 gave at least 90% reduction in growth of one or more weed species.

According to a further feature of the present invention there provides pesticidally active compounds and methods of use of said compounds for the control of a number of pest species which includes: arthropods, especially insects. The compounds thus are advantageously employed in practical uses, for example, in agricultural or horticultural crops, forestry, veterinary medicine or livestock husbandry, or in public health.

A feature of the present invention therefore provides a method of control of pests at a locus which comprises the treatment of the locus (e.g., by application or administration) with an effective amount of a compound of formula I, wherein the substituent groups are as hereinbefore defined. The locus includes, for example, the pest itself or the place (plant, animal, person, field, structure, premises, forest, orchard, waterway, soil, plant or animal product, or the like) where the pest resides or feeds.

The compounds of this invention are preferably useful in the control via foliar application or systemic action of some arthropods, especially some insects, which feed on the above ground portions of plants. Control of foliar pests may additionally be provided by application to the plant roots or plant seeds with subsequent systemic translocation to the above ground portions of the plants.

The compounds of this invention may be useful to control soil insects, such as corn rootworm, termites (especially for protection of structures), root maggots, wireworms, root weevils, stalkborers, cutworms, root aphids, or grubs. They may also be used to provide activity against plant pathogenic nematodes, such as root-knot, cyst, dagger, lesion, or stem or bulb nematodes, or against mites. For the control of soil pests, for example corn rootworm, the compounds are advantageously applied to or incorporated at an effective rate into the soil in which crops are planted or to be planted or to the seeds or growing plant roots.

In the area of public health, the compounds are especially useful in the control of many insects, especially filth flies or other Dipteran pests, such as houseflies, stableflies, soldierflies, hornflies, deerflies, horseflies, midges, punkies, blackflies, or mosquitoes.

Compounds of the invention may be used in the following applications and on pests including arthropods, especially insects or mites, nematodes, or helminth or protozoan pests.

The invention, as previously described, provides methods of control of pests via application or administration of an effective amount of compounds of formula I at a locus which comprises treatment of the locus.

In practical use for the control of arthropods, especially insects or mites, or nematode pests of plants, a method, for example, comprises applying to the plants or to the medium in which they grow an effective amount of a compound of the invention. For such a method, the active compound is generally applied to the locus in which the arthropod or nematode infestation is to be controlled at an effective rate in the range of about 0.005 kg to about 15 kg of the active compound per hectare of locus treated. Under ideal conditions, depending on the pest to be controlled, a lower rate may offer adequate protection. On the other hand, adverse weather conditions, resistance of the pest or other factors may require that the active ingredient be used at higher rates. The optimum rate depends usually upon a number of factors, for example, the type of pest being controlled, the type or the growth stage of the infested plant, the row spacing or also the method of application. More preferably an effective rate range of the active compound is from about 0.01 kg/ha to to about 2 kg/ha.

When a pest is soil-borne, the active compound generally in a formulated composition, is distributed evenly over the area to be treated (ie, for example broadcast or band treatment) in any convenient manner. Application may be made, if desired, to the field or crop-growing area generally or in close proximity to the seed or plant to be protected from attack. The active component can be washed into the soil by spraying with water over the area or can be left to the natural action of rainfall. During or after application, the formulated compound can, if desired, be distributed mechanically in the soil, for example by ploughing, disking, or use of drag chains. Application can be prior to planting, at planting, after planting but before sprouting has taken place, or after sprouting. Additionally, a method of control may also comprise treatment of the seed prior to planting with subsequent control effected after planting the seed.

Methods of control of pests also consist of application to or treatment of the foliage of plants to control arthropods, especially insects or mites, or nematodes attacking the aerial parts of the plants. In addition, methods of control of pests by the invention compounds are provided to control pests which feed on parts of the plant remote from the point of application, e.g., leaf feeding insects which are controlled via systemic action of the active compound when applied for example to the roots of a plant or to the plant seed prior to planting. Furthermore, the compounds of the invention may reduce attacks on a plant by means of antifeeding or repellent effects.

The compounds of the invention and methods of control of pests therewith are of particular value in the protection of field, forage, plantation, glasshouse, orchard or vineyard crops, of ornamentals, or of plantation

or forest trees, for example: cereals (such as maize, wheat, rice, or sorghum), cotton, tobacco, vegetables (such as beans, cole crops, curcurbits, lettuce, onions, tomatoes or peppers), field crops (such as potatoes, sugar beets, ground nuts, soybeans, or oil seed rape), sugar cane, grassland or forage crops (such as maize, sorghum, or lucerne), plantations (such as tea, coffee, cocoa, banana, palm oil, coconut, rubber, or spices), orchards or groves (such as of stone or pit fruit, citrus, kiwifruit, avocado, mango, olives or walnuts), vineyards, ornamental plants, flowers or vegetables or shrubs under glass or in gardens or parks, or forest trees (both deciduous and evergreen) in forests, plantations or nurseries.

They are also valuable in the protection of timber (standing, felled, converted, stored or structural) from attack, for example, by sawflies or beetles or termites.

They have applications in the protection of stored products such as grains, fruits, nuts, spices or tobacco, whether whole, milled or compounded into products, from moth, beetle, mite or grain weevil attack. Also protected are stored animal products such as skins, hair, wool or feathers in natural or converted form (e.g. as carpets or textiles) from moth or beetle attack as well as stored meat, fish or grains from beetle, mite or fly attack.

Additionally, the compounds of the invention and methods of use thereof are of particular value in the control of arthropods, helminths or protozoa which are injurious to, or spread or act as vectors of diseases in man and domestic animals, for example those hereinbefore mentioned, and more especially in the control of ticks, mites, lice, fleas, midges, or biting, nuisance or myiasis flies. The compounds of the invention are particularly useful in controlling arthropods, helminths or protozoa which are present inside domestic host animals or which feed in or on the skin or suck the blood of the animal, for which purpose they may be administered orally, parenterally, percutaneously or topically.

## METHOD OF USE OF INSECTICIDAL COMPOUNDS

The following representative test procedures, using compounds of the invention, were conducted to determine the pesticidal use and activity of compounds of the invention against certain insects, including aphids, two species of caterpillar, a fly and a beetle larvae. The specific species tested were as follows:

| GENUS. SPECIES | COMMON NAME | (ABBREVIATION) |
|---|---|---|
| Aphis nasturtii | Buckthorn aphid | BA |
| Spodoptera eridania | Southern armyworm | SAW |
| Epilachna varivestis | Mexican bean beetle | MBB |
| Musca domestica | Housefly | HF |
| Aphis gossypii | Cotton aphid | CA |
| Heliothis virescens | Tobacco budworm | TBW |

Formulations:

The test compounds were formulated for use according to the following methods.

For aphids, southern armyworm and Mexican bean beetle, a solution or suspension was prepared by adding 10 mg of the test compound to a solution of 160 mg of dimethylformamide, 838 mg of acetone, 2 mg of a 3:1 ratio of Triton X-172 : Triton X-152 (respectively, mainly anionic and nonionic low foam emulsifiers which are each anhydrous blends of alkylaryl polyether alcohols with organic sulfonates), and 98.99 g of water. The result was a concentration of 100 ppm of the test compound.

For housefly tests, the formulation was initially prepared in a similar manner to the above, but in 16.3 g of water with corresponding adjustment of other components, providing a 200 ppm concentration. Final dilution with an equal volume of a 20% by weight aqueous solution of sucrose provided a 100 ppm concentration of the test compound. When necessary, sonication was provided to insure complete dispersion.

For tobacco budworm contact tests, a stock solution was prepared by dissolving the compound in DMF-acetone and then further diluted to provide the required serial dilution concentrations.

Test Procedures:

The above formulated test compounds were then evaluated for their pesticidal activity at the specified concentrations, in ppm (parts per million) by weight, according to the following test procedures:

Buckthorn or cotton aphid: Adult and nymphal stages of buckthorn or cotton aphid were reared on potted dwarf nasturtium or cotton plants, respectively. The potted plants (one pot per compound tested) infested with 100-150 aphids, were placed on a revolving turntable and sprayed with 100 ml of the 100 ppm test compound formulation by use of a DeVilbiss spray gun set at 40 psig air pressure. As an untreated control, 100 ml of a water-acetone-DMF-emulsifiersolution, containing no test compound, were also sprayed on infested plants. A treated control with a commercial technical compound, malathion or cyhalothrin, formulated in the same manner, was tested as a standard. After spraying, the pots were stored for one day for buckthorn aphid or three days for cotton aphid, after which the dead aphids were counted.

Southern armyworm: Potted bean plants, were placed on a revolving turntable and sprayed with 100 ml of the 100 ppm test compound formulation by use of a DeVilbiss spray gun set at 40 psig air pressure. As an untreated control, 100 ml of a water-acetone-DMF-emulsifiersolution, containing no test compound, were also sprayed on plants. A treated control with a commercial technical compound, either cypermethrin or sulprofos, formulated in the same manner, was tested as a standard. When dry, the leaves were placed in plastic cups lined with moistened filter paper. Five randomly selected second instar southern armyworm larvae were introduced into each cup which was closed and held for five days. Larvae which were unable to move the length of the body, even upon stimulation by prodding, were considered dead.

Tobacco budworm: Potted cotton plants were placed on a revolving turntable and sprayed with 100 ml of the 100 ppm test compound formulation by use of a DeVilbiss spray gun set at 40 psig air pressure. As an untreated control, 100 ml of a water-acetone-DMF-emulsifiersolution, containing no test compound, were also sprayed on plants. A treated control with a commercial technical compound, either cypermethrin or sulprofos, formulated in the same manner, was tested as a standard. When dry, the leaves were placed in plastic dishes containing a piece of filter paper and a moistened dental wick. One randomly selected second instar tobacco budworm larva was then introduced into each cup which was closed and held for five days. Larvae unable to move the length of their body, even upon stimulation by prodding, were considered dead.

Mexican bean beetle: Potted bean plants were placed on a revolving turntable and sprayed with 100 ml of the 100 ppm test compound formulation, sufficient to wet the plants to runoff, by use of a DeVilbiss spray gun set at 40 psig air pressure. As an untreated control, 100 ml of a water-acetone-DMF-emulsifier solution, containing no test compound, were also sprayed on plants. A treated control with a commercial technical compound, either cypermethrin or sulprofos, formulated in the same manner, was tested as a standard. When dry, the leaves were placed in plastic cups lined with moistened filter paper. Five randomly selected second instar Mexican bean beetle larvae were introduced into each cup which was closed and held for five days. Larvae which were unable to move the length of the body, even upon stimulation by prodding, were considered dead.

House fly: Four to six day old adult house flies were reared according to the specifications of the Chemical Specialties Manufacturing Association (Blue Book, McNair-Dorland Co., N.Y. 1954; pages 243-244, 261) under controlled conditions. The flies were immobilized by anesthetizing with carbon dioxide and twenty five immobilized individuals, males and females, were transferred to a cage consisting of a standard food strainer and a wrapping-paper-covered surface. Ten ml of the 100 ppm test compound formulation were added to a soufflé cup containing an absorbent cotton pad. As an untreated control, 10 ml of a water-acetone-DMF-emulsifier-sucrose solution, containing no test compound, were applied in a similar manner. A treated control with a commercial technical compound, malathion, formulated in the same manner, was tested as a standard. The bait cup was introduced inside the food strainer prior to admitting the anesthetized flies. Alter 24 hours, flies which showed no sign of movement on stimulation were considered dead.

Use Results: insecticidal activity for some of the representative compounds of the invention are discussed below or the results of some compounds are set forth in **TABLE 1** against the indicated test species (BA/CA, SAW, MBB, HF, TBW: designated by common name abbreviations) and at the indicated dosage rates. Compounds of the invention exhibit reduced or antifeeding properties for some pest species, for example for foliar pests such as southern armyworm and Mexican bean beetle.

In the above discussion and the results reported in **TABLE 1**, representative compounds according to the invention are applied at various concentrations. The use of a 1 ppm (concentration of the compound in parts per million of the test solution applied) foliar solution or suspension or emulsion corresponds approximately to an application of 1 g/ha of active ingredient, based upon an approximate spray volume of 1000 liters/ha (sufficient to run off). Thus applications of foliar sprays of from about 6.25 to 500 ppm would correspond to about 6-500 g/ha.

**TABLE 1**

Use Example of Insecticidal Activity of Phthalazinone Compounds

Foliar on Bait Application

| Example D1 | Conc (ppm) | BA/CA | SAW | MBB | HF | TBW |
|---|---|---|---|---|---|---|
| 5 | 500 | C | C | A | A | - |
| 6 | 500 | C | B | C | C | - |
| 7 | 500 | C | C | A | C | - |
| 8&52 | 250 | C | C | B | C | - |
| 9 | 500 | C | A | A | C | A |
| 10 | 500 | B | C | C | C | - |
| 11 | 500 | C | A | C | C | A |
| 47&48 | 500 | C | C | C | C | - |
| 49 | 500 | C | C | C | C | - |
| 50 | 500 | C | C | C | C | - |
| 51 | 500 | C | C | C | C | - |

A 70 - 100% Mortality

B 30 - 50% Mortality
C 0 - 29% Mortality
\- Not Tested

While the present invention has been set forth in specific and illustrative details and described with preferred particularity, it is susceptible to changes, modifications or alternations, obvious to one of ordinary skill in the art, without departing from the scope and spirit of the invention, which is defined by the claims appended hereto.

**Claims**

1. A method of controlling the growth of pests at a locus which comprises applying to the locus a pesticidally effective amount of a phthalazin-1-one or phthalazin-1-thione derivative of formula I:

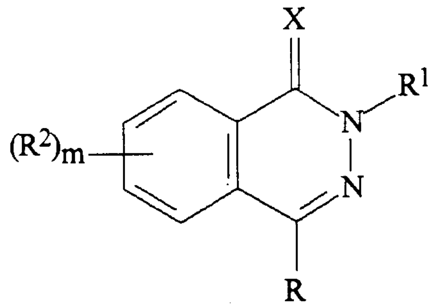

(I)

wherein:

R represents:-

the hydrogen atom;

a straight- or branched chain alkyl, alkenyl or alkynyl group containing up to six carbon atoms optionally substituted by one or more halogen atoms;

a group $-(-CR^3R^4)_n-(phenyl)-(R^{5a})_q$;

a group $-(-CR^3R^4)_nHet$;

a group $-(-CR^3R^4)_n-Ar$, wherein Ar represents phenyl or pyridyl optionally substituted by one or more groups $R^{5a}$ and wherein two substituents on adjacent positions of the ring, together with the two atoms to which they are attached, form a 5- to 7-membered alicyclic ring (which is optionally unsaturated) or an aromatic ring, optionally containing one or more heteroatoms, wherein the alicyclic or aromatic ring is optionally substituted by one or more groups $R^{51}$ which may be the same or different;

$R^1$ represents:-

a straight- or branched chain alkyl, alkenyl or alkynyl group containing up to six carbon atoms optionally substituted by one or more halogen atoms;

a group $-(-CR^3R^4)_n-(phenyl)-(R^{5b})_q$;

a group $-(-CR^3R^4)_nHet$;

a group $-(-CR^3R^4)_n-Ar$, wherein Ar represents phenyl or pyridyl optionally substituted by one or more groups $R^{5b}$ and wherein two substituents on adjacent positions of the ring, together with the two atoms to which they are attached, form a 5- to 7-membered alicyclic ring (which is optionally unsaturated) or an aromatic ring, optionally containing one or more heteroatoms, wherein the alicyctic or aromatic ring is optionally substituted by one or more groups $R^{51}$ which may be the same or different;

provided that R represents a group $-(-CR^3R^4)_n-(phenyl)-(R^{5a})_q$ and/or $R^1$ represents a group $-(-CR^3R^4)_n-(phenyl)-(R^{5b})_q$ wherein n is zero;

$R^2$ represents:-

a group $R^5$;

or phenyl optionally substituted by from one to five groups $R^5$ which may be the same or different;

X represents oxygen or sulphur;

m represents zero or an integer from one to four;

where m is greater than one the groups $R^2$ may be the same or different;

$R^3$ and $R^4$, which may be the same or different, each represents the hydrogen atom or a straight- or branched- chain alkyl group containing up to four carbon atoms optionally substituted by one or more halogen atoms;

$R^5$, $R^{5a}$ and $R^{5b}$, which may be the same or different, each represents:-

a halogen atom;

a straight- or branched- chain alkyl, alkenyl or alkynyl group containing up to six carbon atoms optionally substituted by one or more halogen atoms; or

a group selected from cyano, nitro, $-CO_2R^6$, $-S(O)_pR^6$, $-NR^3R^4$, $-COR^6$, $-S(O)_pR^7$, $-CO_2R^7$, $-OR^7$, $-CONR^3R^4$, $-OSO_2R^7$, $-OSO_2R^8$, $-OCH_2R^7$, $-N(R^3)COR^8$, $-N(R^3)SO_2R^8$, $-N(R^3)SO_2R^7$, $-SO_2NR^3R^4$, $-Si-(R^8)_3$ and $-OR^6$;

n represents zero one or two; where n is two the groups $-(-CR^3R^4)-$ may be the same or different;

q represents zero or an integer from one to five; where q is greater than one the groups $R^{5a}$ and/or

$R^{5b}$ may be the same or different;

$R^{51}$ is as defined above for $R^5$ or represents =O or =S;

Het represents a 5- or 6- membered heterocycle containing from 3 to 5 carbon atoms in the ring and from 1 to 3 heteroatoms in the ring selected from nitrogen, sulphur and oxygen optionally substituted by one or more groups $R^5$ which may be the same or different;

$R^6$ represents the hydrogen atom or a straight- or branched-chain alkyl group containing up to six carbon atoms optionally substituted by one or more halogen atoms;

p represents zero, one or two;

$R^7$ represents phenyl optionally substituted by from one to five groups which may be the same or different selected from halogen, nitro, cyano, $R^6$ and $-OR^6$;

$R^8$ represents a straight- or branched- chain alkyl group containing up to six carbon atoms optionally substituted by one or more halogen atoms; or an agriculturally acceptable salt thereof.

**2.** A compound of formula (I) as defined in claim 1 with the exclusion of the following compounds:

(a) X is oxygen, m is zero, $R^1$ is phenyl and R is 4-$N(CH_3)_2$-phenyl;

(b) X is oxygen, R is alkyl and $(R^2)_m$ is 6,8-dimethyl-7-alkylcarboxy, 5-nitro-6-methyl-7-alkylcarboxy or 5-nitro-6,8-dimethyl-7-alkylcarboxy;

(c) X is oxygen, m is zero, $R^1$ is selected from the group consisting of hydrogen, phenyl, 4-chlorophenyl and 3-chlorophenyl and R is phenyl substituted in the 3,4 or 5- position by $-NH_2$ and optionally bearing one or two additional substituents from alkyl, halogen, alkoxy, hydroxy, phenoxy phenylsulphonamide and nitro;

(d) X is oxygen and $R^1$ is $-(CH_2)_n$Het wherein n is 2;

(e) $R^1$ is alkyl, phenyl or benzyl and R is $-(CR^3R^4)_n$Het;

(f) X is oxygen, m is zero, $R^1$ is 4-pyridylmethyl, benzyl or ethyl and R is phenyl, 3-nitrophenyl, 3-halophenyl, or 3-chloro-5-bromophenyl;

(g) $R^1$ is phenyl or methyl and R is 3-methyl-4-halophenyl;

(h) X is oxygen, m is zero, R is 3,4-dichlorophenyl and $R^1$ is selected from phenyl, methyl and benzyl;

(i) X is oxygen, m is zero, $R^1$ is phenyl, 4-nitrophenyl or 2,4-dinitrophenyl and R is methyl, phenyl, 4-tolyl, 4-chlorophenyl or 4-methoxyphenyl;

(j) m is zero, $R^1$ is phenyl, 2-nitrophenyl or ethyl and R is phenyl, 4-alkylphenyl, 4-halophenyl or 4-nitrophenyl;

(k) X is oxygen, m is zero, R is phenyl and $R^1$ is 4-alkylphenyl, 4-alkoxyphenyl or 4-halophenyl;

(l) X is oxygen, R is 4-halogen-3-methylphenyl and $R^1$ is hydrogen, methyl, phenyl or benzyl;

(m) X is oxygen, m is zero and $R^1$ is $C_{3-6}$ alkyl substituted by a chlorine atom;

(n) X is oxygen, m is zero, R is 4-chlorophenyl and $R^1$ is $-CH_2C{\equiv}CCH_2CH_3$;

(o) R is naphthyl, benzyl or methyl and $R^1$ is phenyl;

(p) R and $R^1$ are phenyl and m is greater than zero;

(q) R is dimethylphenyl and $R^1$ is phenyl, 2,5-dinitrophenyl, 4-methoxyphenyl, or $-CH_2$(N-piperidine);

(r) R is hydrogen or 4-ethylphenyl and $(R^2)_m$ is 6,7-$OMe_2$;

(s) R is phenyl and $R^1$ is $-(-CR^3R^4)_n$Het;

(t) R is 4-tolyl, $R^1$ is methyl or 4-methoxyphenyl and $(R^2)_m$ is tetrabromo;

(u) R is hydrogen and $R^1$ is phenyl, benzyl, or naphthyl;

(v) X is oxygen, R is hydrogen, $R^1$ is 2-tolyl,2-fluorophenyl, 2-methoxyphenyl and $(R^2)_m$ is 7-ethylcarboxy or 7-carboxy;

(w) X is oxygen, R is hydrogen, $R^1$ is trifluoromethylphenyl and $(R^2)_m$ is 6-$NH_2$-7-Cl, 6-$SO_2NH_2$-7-Cl or 6-Cl-7-$SO_2NH_2$;

(x) R is hydrogen and $R^1$ is phenyl substituted in the 4-position by bromine, nitro or methoxy or in the 2-position by fluorine, methyl or methoxy;

(y) X is oxygen, R is hydrogen, $R^1$ is phenyl substituted in the 4-position by carboxy, nitro or fluorine, or in the 2-position by methyl, and $(R^2)_m$ is 8-carboxy or 8-methylcarboxy;

(z) X is oxygen, R is hydrogen, $R^1$ is 4-nitrophenyl and $(R^2)_m$ is 7,8-dimethoxy; or an agriculturally acceptable salt thereof.

**3.** A composition suitable for pesticidal use comprising one or more of the phthalazin-1-one and phthalazin-1-thione derivatives of formula (I) as defined in claim 2 or an agriculturally acceptable salt thereof, in association with one or more compatible agriculturally- acceptable diluents or carriers.

**4.** A method of controlling the growth of weeds at a locus which comprises applying to the locus a herbicidally effective amount of a phthalazin-1-one or phthalazin-1-thione derivative of formula I:

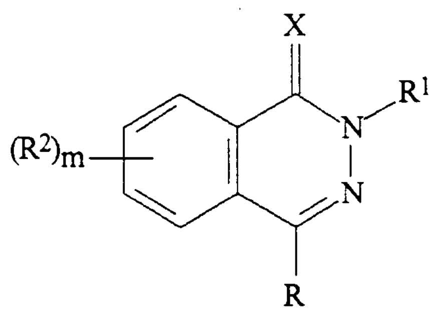

(I)

wherein R represents:-

a straight- or branched chain alkyl, alkenyl or alkynyl group containing from two to six carbon atoms optionally substituted by one or more halogen atoms;

a group $-(-CR^3R^4)_n-(phenyl)-(R^{5a})_q$;

a group $-(-CR^3R^4)_nHet$;

a group $-(CR^3R^4)_n-Ar$, wherein Ar represents phenyl or pyridyl optionally substituted by one or more groups $R^{5a}$ and wherein two substituents on adjacent positions of the ring, together with the two atoms to which they are attached, form a 5- to 7-membered alicyclic ring (which is optionally unsaturated) or an aromatic ring, optionally containing one or more heteroatoms, wherein the alicyclic or aromatic ring is optionally substituted by one or more groups $R^{51}$ which may be the same or different;

$R^1$represents:-

a straight- or branched chain alkyl, alkenyl or alkynyl group containing from two to six carbon atoms optionally substituted by one or more halogen atoms;

a group $-(-CR^3R^4)_n-(phenyl)-(R^{5b})_q$;

a group $-(-CR^3R^4)_nHet$;

a group $-(-CR^3R^4)_n-Ar$, wherein Ar represents phenyl or pyridyl optionally substituted by one or more groups $R^{5b}$ and wherein two substituents on adjacent positions of the ring, together with the two atoms to which they are attached, form a 5- to 7-membered alicyclic ring (which is optionally unsaturated) or an aromatic ring, optionally containing one or more heteroatoms, wherein the alicyclic or aromatic ring is optionally substituted by one or more groups $R^{51}$ which may be the same or different;

provided that R represents a group $-(-CR^3R^4)_n-(phenyl)-(R^{5a})_q$ and /or $R^1$ represents a group $-(-CR^3R^4)_n-(phenyl)-(R^{5b})_q$ wherein n is zero;

and R is not phenyl mono-substituted in the 4-position by halogen;

$R^2$ represents:-

a group $R^5$; or phenyl optionally substituted by from one to five groups $R^5$ which may be the same or different;

X represents oxygen or sulphur;

m represents zero or an integer from one to four;

where m is greater than one the groups $R^2$ may be the same or different;

$R^3$ and $R^4$, which may be the same or different, each represents the hydrogen atom or a straight- or branched- chain alkyl group containing up to four carbon atoms optionally substituted by one or more halogen atoms;

$R^5$, $R^{5a}$ and $R^{5b}$, which may be the same or different, each represents:-

a halogen atom;

a straight- or branched- chain alkyl, alkenyl or alkynyl group containing up to six carbon atoms optionally substituted by one or more halogen atoms; or

a group selected from cyano, nitro, $-CO_2R^6$, $-S(O)_pR^6$, $-NR^3R^4$, $-COR^6$, $-S(O)_pR^7$, $-CO_2R^7$, $-OR^7$, $-CONR^3R^4$, $-OSO_2R^7$, $-OSO_2R^8$, $-OCH_2R^7$, $-N(R^3)COR^8$, $-N(R^3)SO_2R^8$, $-N(R^3)SO_2R^7$, $-SO_2NR^3R^4$, $-Si-(R^8)_3$ and $-OR^6$;

n represents zero, one or two; where n is two the groups $-(-CR^3R^4)-$ may be the same or different;

q is zero or an integer from one to five; where q is greater than one the groups $R^{5a}$ and/or $R^{5b}$ may

be the same or different;

$R^{51}$ is as defined above for $R^5$ or represents =O or =S;

Het represents a 5- or 6- membered heterocycle containing from 3 to 5 carbon atoms in the ring and from 1 to 3 heteroatoms in the ring selected from nitrogen, sulphur and oxygen optionally substituted by one or more groups $R^5$ which may be the same or different;

$R^6$ represents the hydrogen atom or a straight- or branched-chain alkyl group containing up to six carbon atoms optionally substituted by one or more halogen atoms;

p represents zero, one or two; r represents one or two;

$R^7$ represents phenyl optionally substituted by from one to five groups which may be the same or different selected from halogen, nitro, cyano, $R^6$ and $-OR^6$;

$R^8$ represents a straight- or branched- chain alkyl group containing up to six carbon atoms optionally substituted by one or more halogen atoms;

$R^{61}$ and $R^{62}$ independently are hydrogen, halogen or alkyl;

or an agriculturally acceptable salt thereof.

**5.** A method according to claim 4 using a compound of formula (I) in which m is zero, X is oxygen, R represents phenyl or 3-trifluoromethylphenyl and $R^1$ represents phenyl or 4-fluorophenyl.

**6.** A method according to claim 4 or 5 in which the locus is an area used, or to be used, for growing of crops and the compound is applied at an application rate from 0.01 kg to 4.0 kg per hectare.

**7.** A compound of formula (I) as defined in claim 5 with the exclusion of the following compounds:

(a) X is oxygen, m is zero, $R^1$ is phenyl and R is 4-$N(CH_3)_2$-phenyl;

(b) X is oxygen, R is alkyl and $(R^2)_m$ is 6,8-dimethyl-7-alkylcarboxy;

(c) X is oxygen, m is zero, $R^1$ is selected from the group consisting of phenyl, 4-chlorophenyl and 3-chlorophenyl and R is phenyl substituted in the 3,4 or 5- position by $-NH_2$ and optionally bearing one or two additional substituents selected from alkyl, halogen, alkoxy, hydroxy, phenoxy phenylsulphonamide and nitro;

(d) X is oxygen and $R^1$ is $-(CH_2)_n$Het wherein n is 2;

(e) $R^1$ is phenyl and R is $-(CR^3R^4)_n$Het;

(f) X is oxygen, m is zero, $R^1$ is 4-pyridylmethyl, benzyl or ethyl and R is phenyl, 3-nitrophenyl, 3-halophenyl, or 3-chloro-5-bromophenyl;

(g) $R^1$ is phenyl or methyl and R is 3-methyl-4-halophenyl;

(h) X is oxygen, m is zero, R is 3,4-dichlorophenyl and $R^1$ is selected from phenyl, methyl and benzyl;

(i) X is oxygen, m is zero, $R^1$ is phenyl, 4-nitrophenyl or 2,4-dinitrophenyl and R is methyl, phenyl, 4-tolyl or 4-methoxyphenyl;

(j) m is zero, $R^1$ is phenyl, 2-nitrophenyl or ethyl and R is phenyl, 4-alkylphenyl or 4-nitrophenyl;

(k) X is oxygen, m is zero, R is phenyl and $R^1$ is 4-alkylphenyl, 4-alkoxyphenyl or 4-halophenyl;

(l) X is oxygen, R is 4-halogen-3-methylphenyl and $R^1$ is methyl, phenyl or benzyl;

(m) X is oxygen, m is zero and $R^1$ is $C_{3-6}$ alkyl substituted by a chlorine atom;

(n) R is naphthyl, benzyl or methyl and $R^1$ is phenyl;

(o) R and $R^1$ are phenyl and m is greater than zero;

(p) R is dimethylphenyl and $R^1$ is phenyl, 2,5-dinitrophenyl, 4-methoxyphenyl, or $-CH_2$(N-piperidine);

(q) R is phenyl, $R^1$ is methyl and $(R^2)_m$ is 6,7-dimethoxy;

(r) R is phenyl and $R^1$ is $-(-CR^3R^4)_n$Het; and

(s) R is 4-tolyl, $R^1$ is methyl or 4-methoxyphenyl and $(R^2)_m$ is tetrabromo;

or an agriculturally acceptable salt thereof.

**8.** A compound according to claim 7 wherein:

R represents a group $-(-CR^3R^4-)_n$-(phenyl)-$(R^{5a})_q$;

$R^1$ represents a group $-(-CR^3R^4-)_n$-(phenyl)-$(R^{5b})_q$;

$R^2$ represents:-

a halogen atom;

a straight- or branched- chain alkyl, alkenyl or alkynyl group containing up to six carbon atoms optionally substituted by one or more halogen atoms; or

a group selected from cyano, nitro, $-CO_2R^6$, $-S(O)_pR^6$, $-NR^3R^4$, $-COR^6$, $-S(O)_pR^7$, $-CO_2R^7$, $-OR^7$, $-CONR^3R^4$, $-OSO_2R^7$ and $-OR^6$;

and X represents oxygen.

**9.** A compound according to claim 7 or 8 where n is zero.

**10.** A compound according to claim 7,8 or 9 wherein:
R represents phenyl substituted in the 3-position by $R^{5a}$;
$R^1$ represents phenyl substituted in the 3- and/or 4- position by a group or groups $R^{5b}$;
$R^2$ represents:-
a halogen atom;
a straight- or branched- chain alkyl, alkenyl or alkynyl group containing up to six carbon atoms optionally substituted by one or more halogen atoms;
$R^{5a}$ represents:-
a halogen atom;
a straight- or branched- chain alkyl or alkoxy group containing up to four carbon atoms optionally substituted by one or more halogen atoms;
or a cyano group;
$R^{5b}$ represents:-
a halogen atom;
a straight- or branched- chain alkyl group containing up to four carbon atoms optionally substituted by one or more halogen atoms;
or a cyano group;
m represents zero, one or two; and
X represents oxygen.

**11.** A compound according to claim 7,8 or 9 wherein:
R represents methylenedioxyphenyl, wherein the methylene group is optionally substituted by one or two groups $R^{51}$ which may be the same or different;
$R^1$ represents phenyl optionally substituted in the 3- and/or 4-position by $R^{5b}$;
$R^2$ represents:-
a halogen atom;
a straight- or branched chain alkyl group containing up to four carbon atoms optionally substituted by one or more halogen atoms;
or a group selected from $-S(O)_pR^6$ and $-OR^6$ and $-NR^3R^4$;
$R^{5b}$ represents:-
a halogen atom;
a straight- or branched chain alkyl group containing up to four carbon atoms optionally substituted by one or more halogen atoms;
or a cyano group;
m represents zero, one or two; and X represents oxygen.

**12.** A compound according to any one of claims 7 to 11 in which $R^{51}$ represents halogen.

**13.** A compound according to claim 7 wherein:
R represents:-
phenyl substituted in the 3-position by a group $R^{5a}$;
or 2,3-methylenedioxyphenyl wherein the methylene group is optionally substituted by one or two groups $R^{51}$ which may be the same or different;
$R^1$ represents phenyl substituted in the 3- and/or 4- position by $R^{5b}$;
$R^2$ represents:-
a halogen atom;
a straight or branched chain alky group containing one to three carbon atoms;
$R^{5a}$ represents:-
an alkoxy group containing up to three carbon atoms substituted by from one to seven chlorine or fluorine atoms;
or 2,3-difluoromethylenedioxyphenyl;
$R^{5b}$ represents:-
a halogen atom;
a methyl group substituted by one to three halogens;

or a cyano group;
m represents zero, one or two;
and X represents oxygen.

14. A compound according to any one of claims 7 to 13 in which m is one or two and the group or groups $R^2$ are in the 6-and/or 7-position of the phthalazinone ring.

15. A compound according to claim 14 wherein m is one, $R^2$ is methyl and $R^2$ occupies the 7-position of the phthalazinone ring.

16. A compound according to claim 7 in which R is phenyl monosubstituted in the 3-position by trifluoromethoxy.

17. A compound according to claim 7 which is:
2-(2,4-difluorophenyl)-4-(3-trifluoromethylphenyl)phthalazin-1-one,
2-(4-fluorophenyl)-4-(3-trifluoromethoxyphenyl)phthalazin-1-one,
4-(3-trifluoromethoxyphenyl)-2-(4-trifluoromethylphenyl)phthalazin-1-one,
4-(2,3-difluoromethylenedioxyphenyl)-2-(4-fluorophenyl)phthalazin-1-one.
8-fluoro-2-(4-fluorophenyl)-4-(3-trifluoromethoxyphenyl)phthalazin-1-one,
2-(4-fluorophenyl)-7-methyl-4-(3-trifluoromethoxyphenyl)phthalazin-1-one,
4-(2,3-difluoromethylenedioxyphenyl)-2-(4-trifluoromethylphenyl)phthalazine-1-one,
2-(4-chlorophenyl)-4-(2,3-difluoromethylenedioxyphenyl)phthalazin-1-one,
2-(4-chlorophenyl)-4-(3-trifluoromethylphenyl)phthalazin-1-one,
7-chloro-2-(4-fluorophenyl)-4-(3-trifluoromethoxyphenyl)phthalazin-1-one,
2-(4-fluorophenyl)-7-methyl-4-(3-trifluoromethoxyphenyl)phthalazin-1-one,
2-(4-chlorophenyl)-4-(3-trifluoromethoxyphenyl)phthalazin-1-one,
4-(2,3-difluoromethylenedioxyphenyl)-2-(4-fluorophenyl)-7-methylphthalazin-1-one,
7-chloro-4-(2,3-difluoromethylenedioxyphenyl)-2-(4-fluorophenyl)phthalazin-1-one,
4-(2,3-difluoromethylenedioxyphenyl)-7-methyl-2-(4-trifluoromethylphenyl)phthalazin-1-one,
4-(2,3-difluoromethylenedioxyphenyl)-2-(5-trifluoromethylpyrid-2-yl)phthalazin-1-one,
7-methyl-4-(3-trifluoromethoxyphenyl)-2-(4-trifluoromethylphenyl)phthalazin-1-one,
7-fluoro-2-(4-fluorophenyl)-4-(3-trifluoromethylphenyl)phthalazin-1-one,
2-(4-fluorophenyl)-7-methoxy-4-(3-trifluoromethoxyphenyl)phthalazin-1-one,
6-fluoro-2-(4-fluorophenyl)-4-(3-trifluoromethoxyphenyl)phthalazin-1-one,
7-methoxy-4-(3-trifluoromethoxyphenyl)-2-(4-trifluoromethylphenyl)phthalazin-1-one,
7-chloro-4-(3-trifluoromethoxyphenyl)-2-(4-trifluoromethylphenyl)phthalazin-1-one,
2-(4-fluorophenyl)-4-(3-trifluoromethoxyphenyl)-7-trifluoromethoxyphthalazin-1-one,
2-(4-fluorophenyl)-4-(3-trifluoromethoxyphenyl)-7-trifluoromethylphthalazin-1-one,
7-fluoro-4-(3-trifluoromethoxyphenyl)-2-(4-fluorophenyl)phthalazin-1-one,
7-chloro-4-(2,3-difluoromethylenedioxyphenyl)-2-(4-trifluoromethylphenyl)phthalazin-1-one,
4-(2,3-difluoromethylenedioxyphenyl)-7-fluoro-2-(4-fluorophenyl)phthalazin-1-one,
4-(2,3-difluoromethylenedioxyphenyl)-7-fluoro-2-(4-trifluoromethylphenyl)phthalazin-1-one,
5-fluoro-2-(4-fluorophenyl)-4-(3-trifluoromethoxyphenyl)phthalazin-1-one,
2-(4-fluorophenyl)-4-(pyrid-3-yl)phthalazin-1-one,
4-(3-ethylphenyl)-2-(4-fluorophenyl)phthalazin-1-one,
2-(4-fluorophenyl)-7-nitro-4-(3-trifluoromethoxyphenyl)phthalazin-1-one,
2-(4-fluorophenyl)-6-nitro-4-(3-trifluoromethoxyphenyl)phthalazin-1-one,
6-amino-2-(4-fluorophenyl)-4-(3-trifluoromethoxyphenyl)phthalazin-1-one,
7-amino-2-(4-fluorophenyl)-4-(3-trifluoromethoxyphenyl)phthalazin-1-one,
2-(4-fluorophenyl)-4-(3-trifluoromethylphenyl)-phthalazin-1-one,
or 2-phenyl-4-(3-trifluoromethylphenyl)phthalazin-1-one,
or an agriculturally acceptable salt thereof.

18. A herbicidal composition comprising as active ingredient a herbicidally effective amount of a compound of formula (I) as defined in any one of claims 7 to 17 or an agriculturally acceptable salt thereof, in association with one or more compatible agriculturally- acceptable diluents or carriers.

**19.** A composition according to claim 18 comprising from about 0.05% to about 95% by weight of active ingredient and from about 0.05 to about 25% by weight of surface active agent.

**20.** A composition according to claim 18 or 19 which is in the form of an aqueous suspension concentrate, a wettable powder, a soluble powder, a liquid water soluble concentrate, a liquid emulsifiable suspension concentrate, a granule or an emulsifiable concentrate.

**21.** A method of control of pests at a locus which comprises the treatment of the locus with an effective amount of a phthalazin-1-one or phthalazin-1-thione derivative of formula I

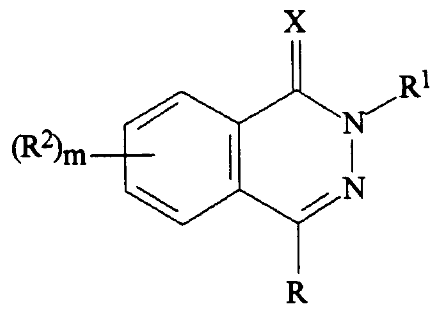

(I)

wherein:
R represents:-
the hydrogen atom; or
phenyl optionally bearing from 1 to 5 substituents selected from the group consisting of halogen, alkyl, haloalkyl, cyano, alkoxy and haloalkoxy;
$R^1$ represents phenyl optionally bearing from 1 to 5 substituents selected from the group consisting of halogen, alkyl, haloalkyl, nitro, alkoxy, haloalkoxy and cyano;
$R^2$ represents:-
a member of the group consisting of halogen, alkyl, haloalkyl, nitro, alkoxy, haloalkoxy and cyano;
X represents oxygen or sulphur; and
m is zero or an integer from 1 to 4;
or an agriculturally acceptable salt thereof.

**22.** A method according to claim 21 for the control of arthropods.

**23.** A method according to claim 21 or 22 wherein from about 0.005 kg to about 15 kg of the active compound per hectare is applied to the locus.

**24.** A compound of formula (I) as defined in claim 21 with the exclusion of the following compounds:
(a) X is oxygen, m is zero, $R^1$ is phenyl and R is 4-$N(CH_3)_2$-phenyl;
(b) $R^1$ is phenyl and R is 3-methyl-4-halophenyl;
(c) X is oxygen, m is zero, R is 3,4-dichlorophenyl and $R^1$ is phenyl;
(d) m is zero, $R^1$ is phenyl, 4-nitrophenyl or 2,4-dinitrophenyl and R is phenyl, 4-tolyl, 4-chlorophenyl or 4-methoxyphenyl;
(e) m is zero, $R^1$ is phenyl or 2-nitrophenyl and R is phenyl, 4-alkylphenyl or 4-halophenyl;
(f) X is oxygen, m is zero, R is phenyl and $R^1$ is 4-alkylphenyl, 4-alkoxyphenyl or 4-halophenyl;
(g) X is oxygen, R is 4-halogen-3-methylphenyl and $R^1$ is phenyl;
(h) R and $R^1$ are phenyl and m is greater than zero;
(i) R is dimethylphenyl and $R^1$ is phenyl, 2,5-dinitrophenyl or 4-methoxyphenyl;
(j) R is hydrogen or phenyl and $(R^2)_m$ is 6,7-dimethoxy;
(k) R is 4-tolyl, $R^1$ is 4-methoxyphenyl and $(R^2)_m$ is tetrabromo;
(l) R is hydrogen and $R^1$ is phenyl;
(m) R is hydrogen and $R^1$ is phenyl substituted in the 4-position by bromine, nitro or methoxy or in the 2-position by fluorine, methyl or methoxy; and

(n) X is oxygen, R is hydrogen, $R^1$ is 4-nitrophenyl and $(R^2)_m$ is 7,8-dimethoxy.

**25.** A compound according to claim 24 wherein R represents phenyl optionally monosubstituted in the 4-position.

**26.** A compound according to claim 24 wherein R is phenyl substituted by 1 to 3 halogen atoms.

**27.** A compound according to claim 24, 25 or 26 wherein $R^1$ is phenyl substituted by one or two substituents selected from the group consisting of nitro, halogen, haloalkyl and haloalkoxy.

**28.** A compound according to any one of claims 24 to 27 in which $R^2$ is in the 6- or 7- position of the phthalazinone ring.

**29.** A compound according to any one of claims 24 to 28 in which $R^2$ is halogen.

**30.** A compound according to any one of claims 24 to 29 in which X is oxygen.

**31.** A compound according to claim 24 wherein m is zero, one or two.

**32.** A compound according to claim 24 wherein:
R represents hydrogen or phenyl bearing halogen;
$R^1$ represents phenyl bearing one or two members of the group consisting of nitro, alkyl, and haloalkoxy;
$R^2$ represents halogen; and
m is zero one or two.

**33.** A compound according to claim 24 wherein R represents hydrogen or phenyl monosubstituted in the 4-position by halogen.

**34.** A compound according to claim 24 which is:
4-(4-bromophenyl)-2-(2,4-dinitrophenyl)phthalazin-1-one,
2-(4-trifluoromethoxyphenyl)phthalazin-1-one,
2-(4-trifluoromethylphenyl)phthalazin-1-one,
2-(4-chlorophenyl)-4-(4-fluorophenyl)phthalazin-1-one,
4-(4-fluorophenyl)-2-(4-trifluoromethyoxyphenyl)phthalazin-1-one,
2-(4-chlorophenyl)-7-fluoro-4-(4-fluorophenyl)phthalazin-1-one,
7-fluoro-4-(4-fluorophenyl)-2-(4-trifluoromethoxyphenyl)phthalazin-1-one,
2-(4-fluorophenyl)phthalazin-1-one,
2-(2,4,6-trichlorophenyl)phthalazin-1-one,
2-(4-chlorophenyl)phthalazin-1-one,
6,7-dichloro-4-(4-chlorophenyl)-2-(4-trifluoromethoxyphenyl)phthalazin-1-one,
6,7-dichloro-2-(4-chlorophenyl)-4-(4-fluorophenyl)phthalazin-1-one,
6,7-dichloro-4-(4-fluorophenyl)-2-(4-trifluoromethoxyphenyl)phthalazin-1-one, or
2-(4-fluorophenyl)-4-(4-fluorophenyl)phthalazin-1-one,
or an agriculturally acceptable salt thereof.

**35.** A pesticidal composition comprising as active ingredient a pesticidally effective amount of at least one compound of formula (I) as defined in any one of claims 24 to 34 or an agriculturally acceptable salt thereof, in association with one or more compatible agriculturally acceptable diluents or carriers.

**36.** A composition according to claim 35 comprising from about 0.05% to about 95% by weight of active ingredient and from about 0.05 to about 25% by weight of surface active agent.

**37.** A composition according to claim 35 or 36 which is in the form of an aqueous suspension concentrate, a wettable powder, a soluble powder, a liquid water soluble concentrate, a liquid emulsifiable suspension concentrate, a granule or an emulsifiable concentrate.

**38.** A process for the preparation of a compound of formula I as defined in claim 1, said process comprising:

(a) reacting a compound of formula II:

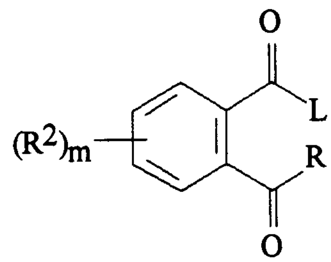

(II)

wherein R, $R^2$ and m are as defined in claim 1 and L is a leaving group, with a hydrazine of formula III or a salt thereof:

$R^1$-$NHNH_2$ (III)

wherein $R^1$ is as defined in claim 1;

(b) where X represents oxygen, cyclising a compound of formula (IIa):

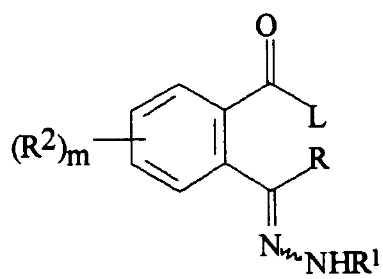

(IIa)

wherein R, $R^1$, $R^2$ and m are as defined in claim 1 and L is as defined above;

(c) where X represents oxygen and R represents $-(-CR^3R^4-)_n$-(phenyl)-$(R^{5a})_q$, $-(-CR^3R^4-)_n$Het or $-(-CR^3R^4-)_n$-Ar, reacting a compound of formula IV:

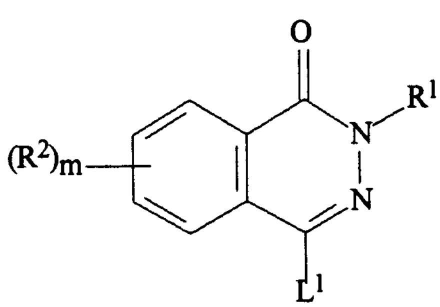

(IV)

wherein $R^1$, $R^2$ and m are as defined in claim 1 and $L^1$ is halogen, with a Grignard reagent of formula V;

R-$MgL^1$ (V)

wherein $L^1$ is as defined above and R represents alkyl, $-(-CR^3R^4)_n$-(phenyl)-$(R^{5a})_q$, $-(-CR^3R^4-)_n$Het or $-(CR^3R^4-)_n$-Ar and $R^3$, $R^4$, $R^{5a}$, q, n, Het and Ar are as defined in claim 1;
(d) where X represents oxygen and R represents alkyl, $-(-CR^3R^4)_n$-(phenyl)-$(R^{5a})_q$, $-(-CR^3R^4-)_n$Het or $-(-CR^3R^4-)_n$-Ar, wherein n is one or two, reacting a compound of formula:

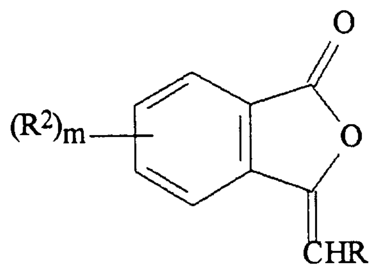

wherein $R^2$ and m are as defined in claim 1 and R is alkyl, $-(-CR^3R^4)_n$-(phenyl)-$(R^{5a})_q$, $-(-CR^3R^4-)_n$Het or $-(-CR^3R^4-)_n$-Ar, wherein n is one or two and $R^3$, $R^4$, $R^{5a}$, q, Het and Ar are as defined in claim 1, with a hydrazine of formula III above $R^1$ is as defined in claim 1 or a salt thereof;
(e) where X represents oxygen and $R^1$ represents alkyl, alkenyl, alkynyl or $-(-CR^3R^4)_n$-(phenyl)-$(R^{5b})_q$, $-(-CR^3R^4-)_n$Het or $-(-CR^3R^4-)_n$-Ar, wherein n is one or two, reacting the corresponding compound of formula I in which $R^1$ is hydrogen with a compound of formula VII:

$R^1$-$L^2$ (VII)

wherein $R^1$ represents alkyl, alkenyl, alkynyl or a group selected from $-(-CR^3R^4)_n$-(phenyl)-$(R^{5b})_q$, $(-CR^3R^4-)_n$Het and $-(-CR^3R^4-)_n$-Ar, wherein n is one or two, $L^2$ is a leaving group and $R^3$, $R^4$, $R^5$, q, Het and Ar are as defined in claim 1;
(f) where X represents sulphur, treating the corresponding compound of formula I in which X represents oxygen with a thionation reagent to convert the carbonyl group to a thiocarbonyl group;
(g) where R is alkenyl or alkynyl, reacting a compound of formula (IV) above wherein $R^1$, $R^2$ and m are as defined in claim 1 and $L^1$ is as defined above with the appropriate alkene or alkyne in the presence of a catalyst;
(h) wherein X is oxygen and R is hydrogen, reacting a compound of formula Va:

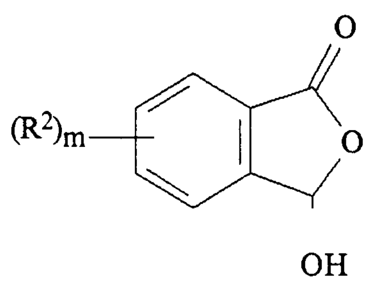

(Va)

wherein $R^2$ and m are as defined in claim 1, with an appropriately substituted hydrazine of formula III above, wherein R is hydrogen, or a salt thereof;

optionally followed by the conversion of the compound of formula I thus obtained into an agriculturally acceptable salt thereof.

**39.** A compound useful as an intermediate in the preparation of a compound of fomula I as defined in claim 1,4 or 20, wherein said compound is of formula:

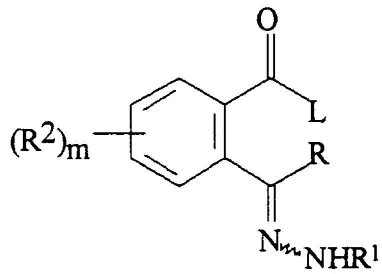

wherein R, $R^1$, $R^2$ and m are as defined in claim 1, 4 or 20 and L represents -OH, straight- or branched- chain alkoxy or halogen.

European Patent Office

# EUROPEAN SEARCH REPORT

Application Number
EP 94 11 0570

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| D,X | GB-A-2 030 136 (MASAYUKI)<br>* the whole document *<br>--- | 2 | C07D237/32<br>C07D237/30<br>C07D405/04<br>A01N43/58<br>C07C251/86 |
| D,X | DE-A-25 31 776 (BASF)<br>* page 4 - page 7 *<br>--- | 2 | |
| D,X | CHEMICAL ABSTRACTS, vol. 111, no. 28, 1989, Columbus, Ohio, US;<br>abstract no. 39285g,<br>S. YAKOVLEV ET AL. 'REACTION OF 2-AROYLBENZOIC ACIDS WITH 4-NITRO- AND 2,4-DINITROPHENYLHYDRAZINE.'<br>page 593 ;column 2 ;<br>* abstract *<br>& ZH. ORG. KHIM.,<br>vol.24, no.11, 1988, RUSS<br>pages 2433 - 2436<br>--- | 34 | |
| X | CHEMICAL ABSTRACTS, vol. 105, no. 5, 1986, Columbus, Ohio, US;<br>abstract no. 42737n,<br>SUGIMOTO,A. ET AL. 'SYNTHESIS AND INHIBITORY EFFECT ON PLATELET AGGREGATION OF 2-PHENYL-1(2H)PHTALAZINONE DERIVATIVES.'<br>page 732 ;<br>* abstract *<br>& CHEM. PHARM. BULL.,<br>vol.33, no.7, 1985, JAPAN<br>pages 2809 - 2820<br>---<br>-/-- | 2 | TECHNICAL FIELDS SEARCHED (Int.Cl.6)<br>C07D<br>C07C |

The present search report has been drawn up for all claims

| Place of search | Date of completion of the search | Examiner |
|---|---|---|
| THE HAGUE | 13 October 1994 | Francois, J |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 94 11 0570

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| D,X | CHEMICAL ABSTRACTS, vol. 83, no. 28, 1975, Columbus, Ohio, US; abstract no. 178862j, FAHMY,A. ET AL. 'ACID AZIDES.III.' page 558 ; * abstract * & INDIAN J. CHEM., vol.13, no.7, 1975 pages 652 - 654 --- | 2 | |
| A | CHEMICAL ABSTRACTS, vol. 110, no. 28, 1989, Columbus, Ohio, US; abstract no. 75534a, page 657 ; * abstract * | 1 | |
| A | & JP-A-63 156 779 (NIPPON SODA) 29 June 1988 --- | 1 | |
| X | FR-A-2 255 289 (BASF) * claims; example 12 * --- | 39 | TECHNICAL FIELDS SEARCHED (Int.Cl.6) |
| X | CHEMICAL ABSTRACTS, vol. 98, no. 23, 1983, Columbus, Ohio, US; abstract no. 198129p, R.BUTLER ET AL. 'OXIDATIONS OF SOME SUBSTITUTED 1,4-BIS(HYDRAZONE) SYSTEMS WITH METALLIC ACETATES.' page 646 ; * abstract * & J.CHEM.RES.SYNOP., no.12, 1982, IRE pages 348 - 349 ----- | 39 | |

The present search report has been drawn up for all claims

| Place of search | Date of completion of the search | Examiner |
|---|---|---|
| THE HAGUE | 13 October 1994 | Francois, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)